# EUROPEAN PATENT APPLICATION

(11) **EP 4 831 077 A1**
(43) Date of publication of application: **28.10.2026**
(21) Application number: 24907473.3
(22) Date of filing: 18.12.2024
(51) Int. Cl.: B65B 55/04, A61L 2/04, A61L 2/10, A61L 2/20, B65B 55/06, B65B 55/10, B65B 55/14, B65B 55/16

(54) **CONTENT FILLING SYSTEM AND METHOD FOR MANUFACTURING CONTENT-FILLED CONTAINER**

(30) Priority: 18.12.2023 JP 2023213269
(71) Applicant: Dai Nippon Printing Co., Ltd., Tokyo 162-8001 (JP)
(72) Inventor: HAYAKAWA Atsushi, Tokyo 162-8001 (JP); SATOU Yoshinori, Tokyo 162-8001 (JP); SEKIGUCHI Hiroki, Tokyo 162-8001 (JP)
(74) Representative: Müller-Boré & Partner Patentanwälte PartG mbB
(86) International application number: PCT/JP2024/044877
(87) International publication number: WO 2025/135089

(57) **Abstract**

A content filling system (10) includes a container sterilization apparatus (20) configured to sterilize a can (1), a lid sterilization apparatus (30) configured to sterilize a lid (2) used to seal the can (1), a water sterilization line (40) configured to sterilize water without heating, a concentrate sterilization line (60) configured to sterilize a product concentrate, a filling apparatus (70) connected to the water sterilization line (40) and the concentrate sterilization line (60) and configured to fill the water and the product concentrate into the sterilized can (1), and a lid attaching apparatus (80) configured to seal, with the sterilized lid (2), the can (1) filled with the water and the product concentrate.

## Description

### Technical Field

The present disclosure relates to a content filling system and a manufacturing method for a contents-filled container.

### Background Art

There is known an aseptic filling system in which sterilized contents are filled into a sterilized container (can) under an aseptic environment, and then the container is sealed with a lid (see, for example, PTL 1).

In such an aseptic filling system, the container is sterilized by spraying a sterilizing agent onto the container inside the aseptic filling system, and then the contents are aseptically filled into the container. In this way, contents-filled containers are manufactured.

In recent years, there has been a demand to reduce carbon dioxide emissions for the purpose of reducing environmental load.

### Citation List

### Patent Literature

PTL 1: Japanese Patent No. 3991571

The present disclosure has been made in view of the above circumstances, and it is an object of the present disclosure to provide a content filling system and a sterilization method capable of reducing carbon dioxide emissions.

### Disclosure of Invention

An embodiment of the present disclosure relates to the following [1] to [32].

[1] A content filling system includes:
   a container sterilization apparatus configured to sterilize a can;
   a lid sterilization apparatus configured to sterilize a lid used to seal the can;
   a water sterilization line configured to sterilize water without heating;
   a concentrate sterilization line configured to sterilize a product concentrate;
   a filling apparatus connected to the water sterilization line and the concentrate sterilization line, the filling apparatus being configured to fill the sterilized can with the water and the product concentrate; and
   a lid attaching apparatus configured to seal, with the sterilized lid, the can filled with the water and the product concentrate.
[2] In the content filling system according to [1], the water sterilization line is configured to sterilize the water by at least one of ultraviolet light and filtration.
[3] In the content filling system according to [1] or [2], the concentrate sterilization line is configured to sterilize the product concentrate by at least one of heating, storage, and filtration.
[4] The content filling system according to any one of [1] to [3] further includes a base alcohol line configured to add alcohol to the sterilized water and the sterilized product concentrate.
[5] In the content filling system according to any one of [1] to [4],
   the container sterilization apparatus includes
   a first sterilizing agent spraying unit configured to spray a sterilizing agent onto the can, and
   a first air rinsing unit configured to air-rinse the can onto which the sterilizing agent has been sprayed by the first sterilizing agent spraying unit.
[6] In the content filling system according to [5],
   the sterilizing agent is a gas or a mist of a hydrogen peroxide aqueous solution, and
   in the first sterilizing agent spraying unit, an amount of the sterilizing agent adhering to the can is greater than or equal to 0.01 µL/cm² and less than or equal to 0.4 µL/cm².
[7] In the content filling system according to [5] or [6],
   the sterilizing agent is a gas or a mist of a hydrogen peroxide aqueous solution,
   in the first air rinsing unit, sterile air at 70°C to 200°C is blown onto the can, and
   after the sterile air has been blown onto the can, an amount of the sterilizing agent adhering to the can is greater than or equal to 0.00001 µL/cm² and less than or equal to 0.01 µL/cm².
[8] In the content filling system according to any one of [5] to [7], the container sterilization apparatus further includes a first heating unit provided upstream of the first sterilizing agent spraying unit, the first heating unit being configured to heat the can.
[9] In the content filling system according to any one of [5] to [8], the container sterilization apparatus further includes a first sterile water rinsing unit provided downstream of the first air rinsing unit.
[10] In the content filling system according to any one of [1] to [9],
   the lid sterilization apparatus includes
   a second sterilizing agent spraying unit configured to spray a sterilizing agent onto the lid, and
   a second air rinsing unit configured to air-rinse the lid onto which the sterilizing agent has been sprayed by the second sterilizing agent spraying unit.
[11] In the content filling system according to [10],
   the sterilizing agent is a gas or a mist of a hydrogen peroxide aqueous solution, and
   in the second sterilizing agent spraying unit, an amount of the sterilizing agent adhering to the lid is greater than or equal to 0.01 µL/cm² and less than or equal to 0.4 µL/cm².
[12] In the content filling system according to [10] or [11],
   the sterilizing agent is a gas or a mist of a hydrogen peroxide aqueous solution,
   in the second air rinsing unit, sterile air at 70°C to 200°C is blown onto the lid, and
   after the sterile air has been blown onto the lid, an amount of the sterilizing agent adhering to the lid is greater than or equal to 0.00001 µL/cm² and less than or equal to 0.01 µL/cm².
[13] In the content filling system according to any one of [10] to [12], the lid sterilization apparatus further includes a second heating unit provided upstream of the second sterilizing agent spraying unit, the second heating unit being configured to heat the lid.
[14] In the content filling system according to any one of [10] to [13], the lid sterilization apparatus further includes a second sterile water rinsing unit provided downstream of the second air rinsing unit.
[15] In the content filling system according to any one of [1] to [4],
   the container sterilization apparatus includes
      a first hot water supply unit configured to supply hot water to the can,
      a temperature of the hot water supplied by the first hot water supply unit is higher than or equal to 60°C and lower than or equal to 100°C, and
   in the first hot water supply unit, an amount of the hot water adhering to the can is greater than or equal to 0.1 mL/cm² and less than or equal to 0.3 mL/cm².
[16] In the content filling system according to [15],
   the container sterilization apparatus includes
   a first air rinsing unit configured to air-rinse the can to which the hot water has been supplied by the first hot water supply unit.
[17] In the content filling system according to any one of [1] to [9],
   the lid sterilization apparatus includes
   a second hot water supply unit configured to supply hot water to the lid,
   a temperature of the hot water supplied by the second hot water supply unit is higher than or equal to 60°C and lower than or equal to 100°C, and
   in the second hot water supply unit, an amount of the hot water adhering to the lid is greater than or equal to 0.1 mL/cm² and less than or equal to 0.3 mL/cm².
[18] In the content filling system according to [17],
   the lid sterilization apparatus includes
   a second air rinsing unit configured to air-rinse the lid to which the hot water has been supplied by the second hot water supply unit.
[19] In the content filling system according to any one of [1] to [18], the can is a seamless can.
[20] In the content filling system according to any one of [1] to [19], the can has a body portion and a bottom portion, and at least one of a recessed portion and a protruding portion is formed in the body portion.
[21] In the content filling system according to any one of [1] to [20], the can has a flange portion, a neck portion, a body portion, and a bottom portion, and the neck portion is a smooth neck.
[22] In the content filling system according to any one of [1] to [21], the can is configured to change in shape due to a pressure change upon opening.
[23] In the content filling system according to any one of [1] to [22], the lid includes aluminum, and a weight of the lid is less than or equal to 3.2 g.
[24] In the content filling system according to any one of [1] to [23], contents to be filled by the content filling system are an acidic carbonated beverage containing carbon dioxide gas.
[25] In the content filling system according to [24], Brix of the contents is higher than or equal to 0% and lower than or equal to 30%.
[26] In the content filling system according to [24] or [25], the contents include fruit juice.
[27] In the content filling system according to any one of [1] to [26], a gas volume in the can sealed with the lid is greater than or equal to 3.0.
[28] The content filling system according to any one of [1] to [27] further includes
   a control unit configured to control the water sterilization line, wherein
   the control unit is configured to clean the lid attaching apparatus with water that has been sterilized without heating by the water sterilization line.
[29] In the content filling system according to any one of [1] to [28], the can includes aluminum, a weight of the can is less than or equal to 11.0 g when a capacity of the can is 350 mL, and a weight of the can is less than or equal to 14.5 g when a capacity of the can is 500 mL.
[30] A manufacturing method for a contents-filled container includes:
   a can sterilization step of performing a sterilization treatment on a can by a container sterilization apparatus;
   a lid sterilization step of performing a sterilization treatment on a lid by a lid sterilization apparatus;
   a water sterilization step of sterilizing water without heating by a water sterilization line;
   a product concentrate sterilization step of sterilizing a product concentrate by a concentrate sterilization line;
   a filling step of filling the water and the product concentrate into the sterilized can by a filling apparatus; and
   a lid attaching step of, by a lid attaching apparatus, sealing, with the sterilized lid, the can filled with the water and the product concentrate.
[31] The manufacturing method for a contents-filled container according to [30] further includes
   a product can sterilization step of sterilizing the can sealed with the lid, wherein
   in the product can sterilization step, a sterilization time is longer than or equal to 0.5 minutes and shorter than or equal to 10 minutes, and a sterilization temperature is higher than or equal to 50°C and lower than or equal to 60°C.
[32] The manufacturing method for a contents-filled container according to [30] or [31] further includes
   a cleaning step of cleaning the lid attaching apparatus after the lid attaching step, wherein
   in the cleaning step, the lid attaching apparatus is cleaned with water that has been sterilized without heating by the water sterilization line.

According to the present disclosure, carbon dioxide emissions from the content filling system can be reduced.

### Brief Description of Drawings

[Fig. 1] Fig. 1 is a schematic plan view showing a content filling system according to an embodiment.
[Fig. 2] Fig. 2 is a schematic view showing a container sterilization apparatus of the content filling system according to the embodiment.
[Fig. 3A] Fig. 3A is a schematic diagram showing a water sterilization line of the content filling system according to the embodiment.
[Fig. 3B] Fig. 3B is a schematic diagram showing another example of the water sterilization line of the content filling system according to the embodiment.
[Fig. 4A] Fig. 4A is a schematic diagram showing a concentrate sterilization line of the content filling system according to the embodiment.
[Fig. 4B] Fig. 4B is a schematic diagram showing another example of the concentrate sterilization line of the content filling system according to the embodiment.
[Fig. 4C] Fig. 4C is a schematic diagram showing another example of the concentrate sterilization line of the content filling system according to the embodiment.
[Fig. 5] Fig. 5 is a schematic view showing a lid sterilization apparatus of the content filling system according to the embodiment.
[Fig. 6] Fig. 6 is a flowchart showing a content filling method using the content filling system according to the embodiment.
[Fig. 7] Fig. 7 is a flowchart showing the content filling method using the content filling system according to the embodiment.
[Fig. 8] Fig. 8 is a schematic plan view showing a modification of the content filling system according to the embodiment.
[Fig. 9] Fig. 9 is a schematic view showing a modification of the container sterilization apparatus of the content filling system according to the embodiment.
[Fig. 10] Fig. 10 is a schematic view showing a modification of the lid sterilization apparatus of the content filling system according to the embodiment.
[Fig. 11] Fig. 11 is a schematic diagram showing a modification of the lid sterilization apparatus of the content filling system according to the embodiment.
[Fig. 12] Fig. 12 is a perspective view showing a can used in the content filling system according to the embodiment.
[Fig. 13] Fig. 13 is a perspective view showing a can used in the content filling system according to the embodiment.
[Fig. 14] Fig. 14 is a perspective view showing a can used in the content filling system according to the embodiment.
[Fig. 15] Fig. 15 is a perspective view showing a can used in the content filling system according to the embodiment.
[Fig. 16] Fig. 16 is a cross-sectional view (cross-sectional view taken along the line XVI,XVII-XVI,XVII in Fig. 15) showing a can used in the content filling system according to the embodiment, and is a cross-sectional view showing the can in an unopened state.
[Fig. 17] Fig. 17 is a cross-sectional view (cross-sectional view taken along the line XVI,XVII-XVI,XVII in Fig. 15) showing a can used in the content filling system according to the embodiment, and is a cross-sectional view showing the can in an opened state.
[Fig. 18] Fig. 18 is a flowchart showing a modification of the content filling method using the content filling system according to the embodiment.
[Fig. 19] Fig. 19 is a flowchart showing a modification of the content filling method using the content filling system according to the embodiment.
[Fig. 20] Fig. 20 is a schematic view showing a modification of a lid attaching apparatus of the content filling system according to the embodiment.
[Fig. 21] Fig. 21 is a schematic plan view showing a modification of a lid attaching chamber of the content filling system according to the embodiment.
[Fig. 22] Fig. 22 is a schematic diagram showing a modification of the lid sterilization apparatus of the content filling system according to the embodiment.

### Description of Embodiments

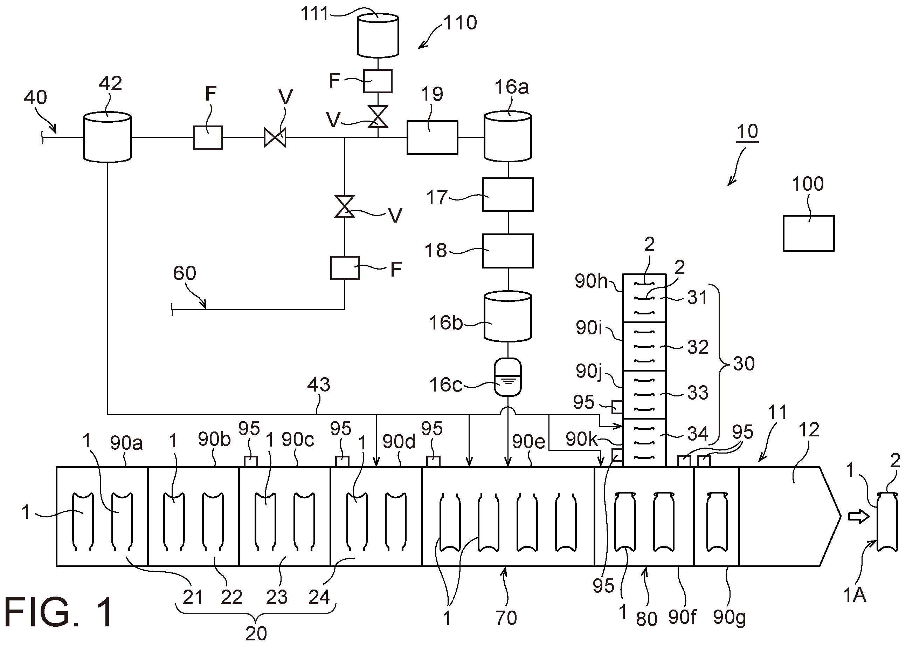

Hereinafter, an embodiment of the present disclosure will be described with reference to the drawings. Figs. 1 to 7 are views showing the embodiment. The drawings described below are schematically shown drawings. Therefore, the size and shape of each portion are exaggerated as needed for the purpose of easy understanding. The embodiment may be modified as needed without departing from the technical idea. In the drawings described below, like reference signs denote the same portions, and the detailed description may be partially omitted. The numeric values, such as dimensions, and material names of members described in the specification are illustrative of the embodiment and are not limited thereto. The numeric values and material names may be selected and used as needed. In the specification, terms that specify shapes and geometrical conditions, for example, terms, such as parallel, orthogonal, and perpendicular, are interpreted as not only the strict sense of the terms but also substantially the same state.

### (Content Filling System)

First, a content filling system (aseptic filling system) according to the embodiment will be described with reference to Fig. 1.

The content filling system 10 shown in Fig. 1 is a system that fills a can (container) 1 with contents, such as a beverage. The contents are prepared by diluting a product concentrate with water. In this case, the product concentrate may be diluted with water by a factor greater than or equal to 1.1 times and less than or equal to 1000 times, and preferably by a factor greater than or equal to 2 times and less than or equal to 10 times. The product concentrate may be diluted with water by a factor greater than or equal to 10 times and less than or equal to 80 times, may be diluted with water by a factor greater than or equal to 20 times and less than or equal to 70 times, or may be diluted with water by a factor greater than or equal to 30 times and less than or equal to 50 times.

The can 1 may be an aluminum can made of aluminum. When the can 1 is an aluminum can, the weight of the can 1 can be reduced.

When the can 1 has a capacity of 350 mL, the weight of the can 1 may be less than or equal to 12.0 g, may be less than or equal to 11.0 g, may be less than or equal to 10.5 g, may be less than or equal to 10.0 g, may be less than or equal to 9.5 g, or may be less than or equal to 9.0 g. When the can 1 has a capacity of 500 mL, the weight of the can 1 may be less than or equal to 15.0 g, may be less than or equal to 14.5 g, may be less than or equal to 14.0 g, may be less than or equal to 13.5 g, may be less than or equal to 13.0 g, may be less than or equal to 12.5 g, may be less than or equal to 12.0 g, or may be less than or equal to 11.0 g. When the can 1 is made of the same material, the manufacturing cost of the can 1 can be reduced as the weight of the can 1 decreases. On the other hand, as the weight of the can decreases, the can becomes more susceptible to deformation. For this reason, when the weight of the can is reduced, the can may be deformed due to pressure changes during heat sterilization or the like. According to the present embodiment, since sterilization of the contents is performed before filling, a sterilization time after filling can be shortened or sterilization after filling can be omitted. Therefore, by using the content filling system 10 according to the present embodiment, deformation can be suppressed even when the weight of the can 1 is reduced.

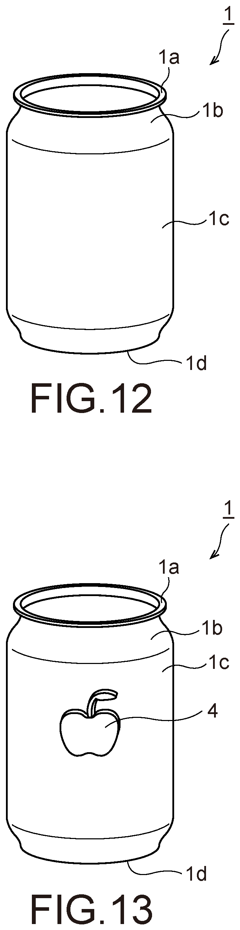

As shown in Fig. 12, the can 1 includes a flange 1a, a neck portion 1b located below the flange 1a, a body portion 1c located below the neck portion 1b, and a bottom portion 1d located below the body portion 1c. The can 1 may be a seamless can. A seamless can is a can formed by drawing and ironing, or the like. By using a seamless can, the body portion 1c of the can 1 can be made thinner, so the weight can be reduced. However, when the weight of the can 1 is reduced, the can 1 may be deformed during a sterilization step of sterilizing a product can 1A (described later) in an existing filling system. According to the present embodiment, since sterilization of the contents is performed before filling, a sterilization time after filling can be shortened or sterilization after filling can be omitted. Therefore, by using the content filling system 10 according to the present embodiment, deformation can be suppressed even in a seamless can in which the body portion 1c of the can 1 is made thinner.

The neck portion 1b of the can 1 may be a smooth neck. By forming the neck portion 1b as a smooth neck, the weight of the can 1 can be reduced. When the weight of the can 1 is reduced, the can 1 generally becomes more susceptible to pressure, so the can 1 may be deformed during heat sterilization or the like in the existing filling system. According to the present embodiment, since sterilization of the contents is performed before filling, a sterilization time after filling can be shortened or sterilization after filling can be omitted. Therefore, by using the content filling system 10 according to the present embodiment, deformation can be suppressed even when the neck portion of the can 1 is a smooth neck. A smooth neck means that no step portion is formed in the neck portion 1b and the diameter of the neck portion 1b gradually decreases toward the flange 1a.

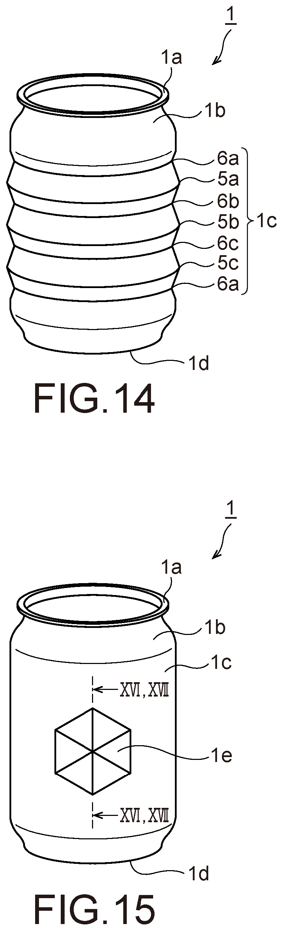

As shown in Figs. 13 and 14, at least one of a recessed portion and a protruding portion may be formed on the body portion 1c of the can 1. In the example shown in Fig. 13, a protruding portion 4 having an apple shape is formed on the body portion 1c of the can 1. In the example shown in Fig. 14, the body portion 1c of the can 1 includes a plurality of large-diameter portions (protruding portions) 5a to 5c and a plurality of small-diameter portions (recessed portions) 6a to 6d. The large-diameter portions 5a to 5c and the small-diameter portions 6a to 6d are alternately arranged in a height direction. A can having at least one of a recessed portion and a protruding portion can have excellent designability. However, in a can having a recessed portion or a protruding portion, pressure applied to the body portion may become non-uniform, and the can may be more susceptible to deformation. During heating, pressure is likely to be applied to the can, so the can may be more susceptible to deformation. By using the content filling system 10 according to the present embodiment, a heating time of the product can 1A (described later) can be shortened or a heating time can be omitted, so deformation of the can 1 having excellent designability can be suppressed. Although not shown in the drawing, only a recessed portion may be formed on the body portion 1c of the can 1.

Furthermore, the can 1 may be configured to change in shape due to a pressure change upon opening. A can that changes in shape due to a pressure change can provide a user with a sense of release upon opening. In general, deformation may be likely to occur due to a change in internal pressure during heat sterilization. During heating, pressure is likely to be applied to the can, so the can may be more susceptible to deformation. By using the content filling system 10 according to the present embodiment, a heating time of the product can 1A (described later) can be shortened or a heating time can be omitted, so deformation of the can 1 configured to change in shape due to a pressure change upon opening can be suppressed.

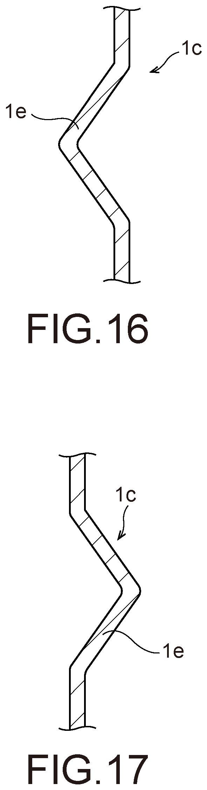

Preferably, a change in shape due to a pressure change is such that the can 1 has a protruding portion due to a high internal pressure when unopened and has a recessed portion due to a decrease in the internal pressure when opened. As a result, since a recessed portion is formed upon opening, a user can use the can that is easily gripped. In this case, for example, as shown in Figs. 15 to 17, a deformable portion 1e may be formed on the body portion 1c of the can 1. Then, as shown in Fig. 16, the deformable portion 1e may protrude radially outward when the can 1 is unopened. On the other hand, as shown in Fig. 17, the deformable portion 1e may be configured to, after the can 1 is opened, be recessed radially inward due to a pressure change upon opening.

As shown in Fig. 1, the content filling system 10 includes a container sterilization apparatus 20, a lid sterilization apparatus 30, a water sterilization line 40, a concentrate sterilization line 60, a filling apparatus (filler) 70, and a lid attaching apparatus (seamer) 80. The content filling system 10 may further include a base alcohol line 110 that adds alcohol to the sterilized water and the sterilized product concentrate. The content filling system 10 may further include a control unit 100 that controls the content filling system 10.

The container sterilization apparatus 20 is an apparatus that sterilizes the can 1. The lid sterilization apparatus 30 is an apparatus that sterilizes the lid 2 used to seal the can 1. The water sterilization line 40 is a line that sterilizes, without heating, water used to dilute a product concentrate. The concentrate sterilization line 60 is a line that sterilizes a product concentrate. The filling apparatus 70 is an apparatus connected to the water sterilization line 40 and the concentrate sterilization line 60, and is an apparatus that fills the sterilized can 1 with water and a product concentrate. The lid attaching apparatus 80 is an apparatus that seals, with the sterilized lid 2, the can 1 filled with water and a product concentrate.

The content filling system 10 includes a product can discharge unit 11 that discharges the can 1 sealed with the lid 2 by the lid attaching apparatus (seamer) 80. In the content filling system 10, the above-described container sterilization apparatus 20, filling apparatus 70, lid attaching apparatus 80, and product can discharge unit 11 are arranged in this order from the upstream side to the downstream side along a conveying direction of the can 1. The content filling system 10 may include a plurality of transfer wheels (not shown) provided between the container sterilization apparatus 20, the filling apparatus 70, the lid attaching apparatus 80, and the like, to convey the can 1 between these apparatuses.

In the present embodiment, the container sterilization apparatus 20 sterilizes the can 1 by spraying a sterilizing agent onto the can 1. By spraying a sterilizing agent, the can 1 is sterilized before being filled with contents.

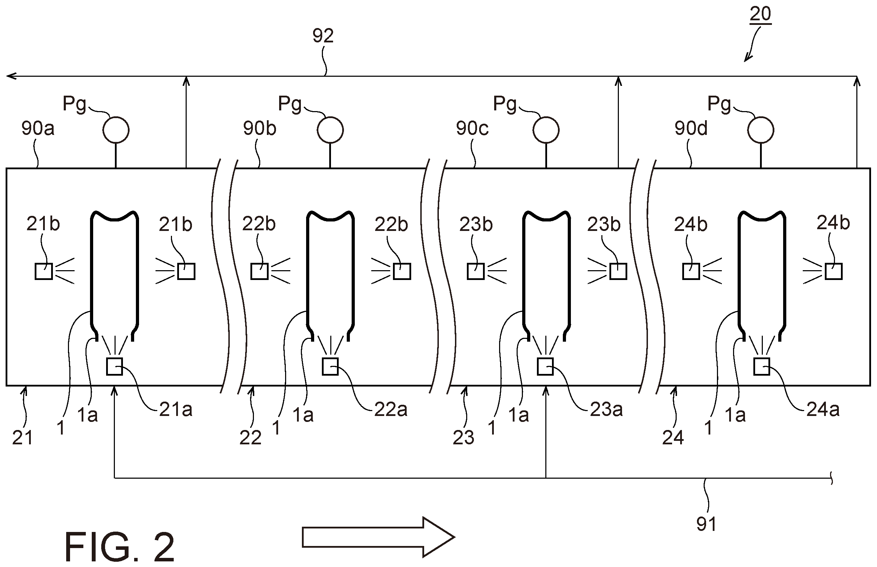

As shown in Figs. 1 and 2, the container sterilization apparatus 20 includes a first sterilizing agent spraying unit 22 that sprays a sterilizing agent onto the can 1, and a first air rinsing unit 23 that air-rinses the can 1 onto which a sterilizing agent has been sprayed by the first sterilizing agent spraying unit 22. The container sterilization apparatus 20 may further include a first heating unit 21 that is provided upstream of the first sterilizing agent spraying unit 22 and that heats the can 1. Furthermore, the container sterilization apparatus 20 may further include a first sterile water rinsing unit 24 provided downstream of the first air rinsing unit 23. In the container sterilization apparatus 20, the first heating unit 21, the first sterilizing agent spraying unit 22, the first air rinsing unit 23, and the first sterile water rinsing unit 24 are arranged in this order from the upstream side to the downstream side along the conveying direction of the can 1. As shown in Fig. 2, the can 1 is conveyed in the direction of the arrow.

The first heating unit 21 is a portion that heats (preheats) the can 1 before a sterilizing agent is sprayed. The first heating unit 21 is configured to heat the can 1 while conveying the can 1. When the can 1 is heated while being conveyed, the can 1 may be conveyed in a state where the flange 1a, to which the lid 2 is to be attached, faces downward. The first heating unit 21 includes first hot air nozzles 21a and 21b that blow heated air onto the can 1. Among these, the first hot air nozzle 21a is a nozzle for blowing heated air onto the inner surface of the can 1. The first hot air nozzles 21b are nozzles for blowing heated air onto the outer surface of the can 1. By heated air blown from the first hot air nozzles 21a and 21b, the can 1 may be, for example, heated such that the surface has a temperature higher than or equal to 40°C and lower than or equal to 100°C or may be heated such that the surface has a temperature higher than or equal to 50°C and lower than or equal to 80°C. When the surface temperature of the can 1 is higher than or equal to 40°C, the sterilization effect on the can 1 can be improved. When the surface temperature of the can 1 is lower than or equal to 100°C, thermal energy consumption can be suppressed, and carbon dioxide emissions can be reduced.

The first sterilizing agent spraying unit 22 is a portion that sterilizes the can 1 by spraying a sterilizing agent onto the can 1 heated by the first heating unit 21. The first sterilizing agent spraying unit 22 is configured to spray a sterilizing agent while conveying the can 1. When a sterilizing agent is sprayed while the can 1 is being conveyed, the can 1 may be conveyed in a state where the flange 1a, to which the lid 2 is to be attached, faces downward. The first sterilizing agent spraying unit 22 includes first spraying nozzles 22a and 22b that spray a sterilizing agent onto the can 1 being conveyed. Among these, the first spraying nozzle 22a is a nozzle for spraying a sterilizing agent onto the inner surface of the can 1. The first spraying nozzles 22b are nozzles for spraying a sterilizing agent onto the outer surface of the can 1. For example, a hydrogen peroxide aqueous solution is used as the sterilizing agent sprayed by the first sterilizing agent spraying unit 22. In the first sterilizing agent spraying unit 22, a gas obtained by vaporizing a hydrogen peroxide aqueous solution at a temperature higher than or equal to the boiling point or a mist in which part of the hydrogen peroxide aqueous solution is liquefied is generated. Then, the gas or the mist is sprayed from the first spraying nozzles 22a and 22b onto the inner and outer surfaces of the can 1. In the first sterilizing agent spraying unit 22, a gas or a mist of a hydrogen peroxide aqueous solution is sprayed onto the inner and outer surfaces of the can 1, so the can 1 is uniformly sterilized. In the present embodiment, hydrogen peroxide is used as a sterilizing agent to sterilize the can 1; however, the sterilizing agent is not limited to hydrogen peroxide, and may be any agent having an effect of inactivating microorganisms, such as peracetic acid and other agents. In addition to the sterilizing agent, hot water or steam (described later) may be used, and these may be used in combination.

In the first sterilizing agent spraying unit 22, when the inner surface of the can 1 is coated with at least one of epoxy resin, polyethylene terephthalate (PET) resin, and polyvinyl chloride, the amount of sterilizing agent adhering to the can 1 may be greater than or equal to 0.01 µL/cm² and less than or equal to 0.4 µL/cm² and may be greater than or equal to 0.03 µL/cm² and less than or equal to 0.1 µL/cm². When the amount of sterilizing agent adhering to the can 1 is greater than or equal to 0.01 µL/cm², the sterilization effect on the can 1 can be improved. When the amount of sterilizing agent adhering to the can 1 is greater than or equal to 0.03 µL/cm², the sterilization effect on the can 1 can be further improved. When the amount of sterilizing agent adhering to the can 1 is less than or equal to 0.4 µL/cm², a residue of the sterilizing agent on the can 1 after passing through the container sterilization apparatus 20 can be suppressed. In this case, the concentration of hydrogen peroxide in the sterilizing agent may be 35 wt%.

The first air rinsing unit 23 is a portion that supplies sterile heated air or room-temperature air to the can 1 onto which a sterilizing agent has been sprayed in the first sterilizing agent spraying unit 22. When sterile heated air or room-temperature air is supplied, hydrogen peroxide is activated, and foreign matter, hydrogen peroxide, and the like, are removed from the inside of the can 1. The first air rinsing unit 23 is configured to supply sterile air while conveying the can 1. In this case, the can 1 may be conveyed in a state where the flange 1a, to which the lid 2 is to be attached, faces downward. By conveying the can 1 in a state where the flange 1a faces downward, foreign matter can be effectively removed from the inside of the can 1. If necessary, hydrogen peroxide gasified by mixing a low-concentration condensed mist of hydrogen peroxide with sterilized air at room temperature may be supplied to the can 1. Sterile air may be air, or may be carbon dioxide or an inert gas.

The first air rinsing unit 23 includes first air rinsing nozzles 23a and 23b that blow sterile air onto the can 1 being conveyed. The first air rinsing nozzle 23a is a nozzle for blowing sterile air onto the inner surface of the can 1. The first air rinsing nozzles 23b are nozzles for blowing sterile air onto the outer surface of the can 1. By blowing sterile air, a gas or a mist of hydrogen peroxide aqueous solution sprayed onto the inner and outer surfaces of the can 1 is uniformly activated. Sterile air may be blown onto only the inner surface of the can 1.

In the first air rinsing unit 23, sterile air at a temperature higher than or equal to 70°C and lower than or equal to 200°C may be blown onto the can 1. For example, the temperature of the sterile air to be blown may be higher than or equal to 80°C, may be higher than or equal to 90°C, may be higher than or equal to 120°C, and may be higher than or equal to 150°C. When the temperature of the sterile air is higher than or equal to 70°C, a sterilizing agent adhering to the can 1 can be effectively activated. When the temperature of the sterile air is lower than or equal to 200°C, carbon dioxide emissions can be reduced. The temperature of the sterile air is a temperature immediately after the sterile air is blown from the first air rinsing nozzles 23a and 23b, that is, a temperature at the tips of the first air rinsing nozzles 23a and 23b.

After sterile air is blown, the amount of sterilizing agent adhering to the can 1 may be greater than or equal to 0.00001 µL/cm² and less than or equal to 0.01 µL/cm² when the inner surface of the can 1 is coated with at least one of epoxy resin, PET resin, and polyvinyl chloride. When the amount of sterilizing agent adhering to the can 1 is greater than or equal to 0.00001 µL/cm², the sterilization effect on the can 1 can be improved. When the amount of sterilizing agent adhering to the can 1 is less than or equal to 0.01 µL/cm², a residue of the sterilizing agent on the can 1 after passing through the container sterilization apparatus 20 can be suppressed.

The first sterile water rinsing unit 24 cleans, with sterile water, the can 1 sterilized by a sterilizing agent (hydrogen peroxide). By cleaning the can 1 with sterile water, an extremely small amount of hydrogen peroxide adhering to the can 1 is washed away, and foreign matter is removed. The first sterile water rinsing unit 24 is configured to supply sterile water while conveying the can 1. In this case, the can 1 may be conveyed in a state where the flange 1a, to which the lid 2 is to be attached, faces downward. By conveying the can 1 in a state where the flange 1a faces downward, a sterilizing agent and foreign matter can be effectively removed from the inside of the can 1. The first sterile water rinsing unit 24 includes first sterile water rinsing nozzles 24a and 24b that spray sterile water onto the can 1 being conveyed. The first sterile water rinsing nozzle 24a is a nozzle for spraying sterile water onto the inner surface of the can 1. The first sterile water rinsing nozzles 24b are nozzles for spraying sterile water onto the outer surface of the can 1. In the first sterile water rinsing unit 24, the temperature of sterile water may be higher than or equal to 5°C and lower than or equal to 100°C. Each of the nozzles 21a, 22a, 23a, and 24a may be inserted into the can to improve efficiency.

Next, the water sterilization line 40, the concentrate sterilization line 60, and the base alcohol line 110 of the content filling system 10 will be described. Initially, the water sterilization line 40 will be described.

The water sterilization line 40 shown in Fig. 1 is a sterilization line that sterilizes water without heating. The water sterilization line 40 may sterilize water by at least one of ultraviolet light and filtration. When the water sterilization line 40 sterilizes water by ultraviolet light, the water in the water sterilization line 40 may be sterilized by ultraviolet light from at least one of a low-pressure mercury lamp or a medium-pressure mercury lamp. When the water sterilization line 40 sterilizes water by filtration, the water sterilization line 40 may sterilize the water by filtering the water with a sterilizing filter (such as a first sterilizing filter 53 or the like (described later)). In the specification, "sterilization without heating" refers to sterilizing water without using thermal energy from an electric heater, steam, or the like.

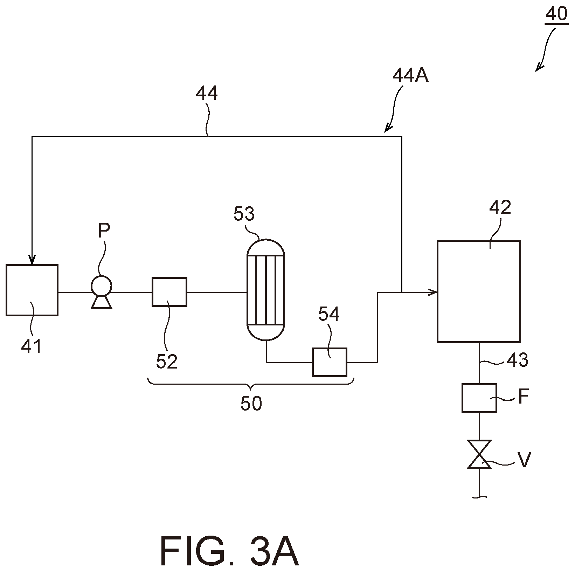

As shown in Fig. 3A, the water sterilization line 40 includes at least a water sterilizer 50 that sterilizes water. In the example shown in Fig. 3A, the water sterilization line 40 includes a first water tank 41, the water sterilizer 50, and a second water tank 42. The first water tank 41, the water sterilizer 50, and the second water tank 42 are arranged in this order from the upstream side to the downstream side along the conveying direction of water. In the water sterilization line 40, a flowmeter F and a sterile valve V with an adjustable opening may be provided downstream of the second water tank 42. Although not shown in the drawing, thermometers, pressure gauges, level gauges, concentration meters, and the like are installed at various locations in the water sterilization line 40.

The first water tank 41 is a so-called balance tank, and serves to smooth the flow of water by storing water. The volume of the first water tank 41 may be larger than or equal to 30 m³ and smaller than or equal to 100 m³ and may be, for example, 50 m³. A pump P for conveying water and a flowmeter (not shown) for measuring the flow rate of water may be provided downstream of the first water tank 41.

The water sterilizer 50 is a sterilizer that sterilizes water stored in the first water tank 41. The details of the water sterilizer 50 will be described later.

The second water tank 42 is a tank (so-called aseptic tank) that stores water sterilized by the water sterilizer 50. The second water tank 42 serves to smooth the flow of water by storing sterilized water. The volume of the second water tank 42 may be larger than or equal to 5 m³ and smaller than or equal to 50 m³ and may be, for example, 10 m³.

A bypass line 43 may be provided downstream of the second water tank 42. As shown in Fig. 1, the bypass line 43 may connect the water sterilization line 40 to the first sterile water rinsing unit 24. The bypass line 43 may connect the water sterilization line 40 to the filling apparatus 70. The bypass line 43 may connect the water sterilization line 40 to the lid attaching apparatus 80. Furthermore, the bypass line 43 may connect the water sterilization line 40 to a second sterile water rinsing unit 34 (described later) of the lid sterilization apparatus 30. When the bypass line 43 is provided, water sterilized by the water sterilizer 50 is used to clean the can 1, the filling apparatus 70, and the lid 2. Since water sterilized by the water sterilizer 50 is used, carbon dioxide emissions from the content filling system 10 can be further reduced as compared with a case where the can 1 and the like are cleaned with sterile water produced by using a sterilizer that sterilizes water by heating.

The bypass line 43 may connect the water sterilization line 40 to chambers 90a to 90k (described later). Then, when the interiors of the chambers 90a to 90k are cleaned, the content filling system 10 may supply water sterilized by the water sterilization line 40 to the chambers 90a to 90k via the bypass line 43. When machines and the like disposed in the chambers 90a to 90k are cleaned, the content filling system 10 may supply water sterilized by the water sterilization line 40 to the chambers 90a to 90k via the bypass line 43. The bypass line 43 may be connected to all supply devices that supply water used in the content filling system 10 during manufacturing of the product can 1A and water used when the product can 1A is not being manufactured.

As shown in Fig. 3A, a circulation line 44 may be connected upstream of the second water tank 42 in the water sterilization line 40. One end of the circulation line 44 may be connected upstream of the second water tank 42, and the other end of the circulation line 44 may be connected to the first water tank 41. Thus, a circulation system 44A for circulating water may be formed by the first water tank 41, the water sterilizer 50, and the circulation line 44. Another tank may be provided between the first water tank 41 and the water sterilizer 50, and the other end of the circulation line 44 may be connected to the other tank. A thermometer (not shown) may be provided on the circulation line 44. A concentration meter (not shown) for measuring the concentration of a sterilizing agent or a cleaning agent when the water sterilizer 50 is sterilized may be provided on the circulation line 44. Furthermore, a heating device (such as a heat exchanger and a heater (not shown)) for heating a sterilizing agent or the like during cleaning and/or sterilization of the circulation line 44 may be installed on the circulation line 44.

Next, the water sterilizer 50 of the water sterilization line 40 will be described. The water sterilizer 50 is a sterilizer that sterilizes water used in the content filling system 10. In the present embodiment, the water sterilizer 50 sterilizes water without heating. As described above, the water sterilizer 50 sterilizes water (pure water) stored in the first water tank 41. Therefore, the water sterilizer 50 sterilizes water having an electric conductivity higher than or equal to 0.1 µS/cm and lower than or equal to 20 µS/cm. Water is preferably pure water; however, water is not limited thereto depending on the type of contents. The water sterilized by the water sterilizer 50 may be ultrapure water, distilled water, purified water, RO water, ion-exchanged water, municipal water, or well water. When the electric conductivity of water is higher than or equal to 20 µS/cm, nitrite nitrogen (or nitrites) may be generated depending on the wavelength and cumulative irradiation dose of ultraviolet light when the water is sterilized with ultraviolet light. To avoid the generation of nitrite nitrogen (or nitrites), a medium-pressure ultraviolet lamp that cuts ultraviolet light shorter than or equal to 220 nm may be used. On the other hand, in Japan, the standard for nitrite nitrogen needs to be lower than or equal to 0.04 mg/L; whereas, in other countries, the standard differs (for example, lower than or equal to 3 mg/L, lower than or equal to 1 mg/L, and lower than or equal to 0.5 mg/L for the WHO, the USEPA, and the EU, respectively). Therefore, when water is sterilized with ultraviolet light, the electric conductivity of the water, or the wavelength and cumulative irradiation dose of the ultraviolet light need to be adjusted and verified such that the amount of nitrite nitrogen does not exceed the standard value of the country where the content filling system 10 is installed. Even if the electric conductivity of the water is 200 µS/cm, the electric conductivity of the water does not necessarily need to be lower than or equal to 20 µS/cm as long as the concentration of nitrite nitrogen in the water after ultraviolet light irradiation is within the standard value.

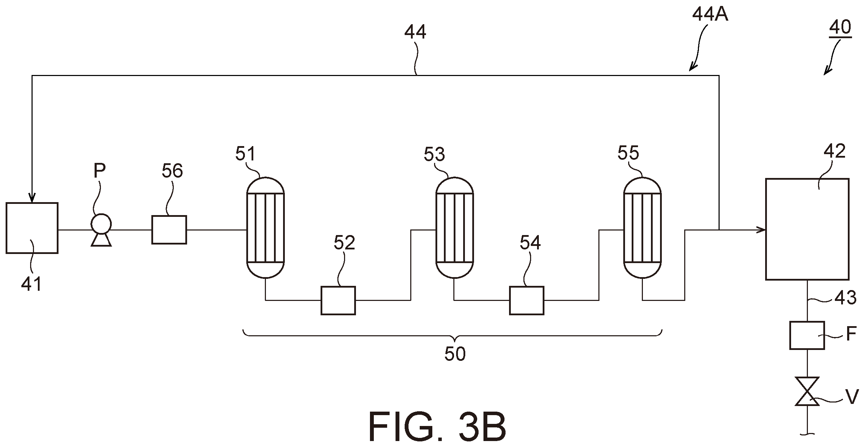

As shown in Figs. 3A and 3B, the water sterilizer 50 includes at least one sterilizing filter (the first sterilizing filter 53 and a second sterilizing filter 55) or at least one sterilizer (a pre-sterilizer 56, a first sterilizer 52, and a second sterilizer 54). Since the water sterilizer 50 includes at least one sterilizing filter or at least one sterilizer, it is possible to produce highly sterile water (sterile quality water needed to fill the product can 1A, which is a final product) while suppressing carbon dioxide emissions.

In the example shown in Fig. 3A, the water sterilizer 50 includes the first sterilizer 52, the first sterilizing filter 53, and the second sterilizer 54. The first sterilizer 52, the first sterilizing filter 53, and the second sterilizer 54 are arranged in this order from the upstream side to the downstream side along the conveying direction of water. In the water sterilizer 50, by disposing the sterilizer (in this case, the second sterilizer 54) downstream of the sterilizing filter (in this case, the first sterilizing filter 53), microorganisms can be sterilized even when the microorganisms pass through the sterilizing filter. Since the water sterilizer 50 includes a plurality of sterilizers (the first sterilizer 52 and the second sterilizer 54), even when one of the sterilizers stops, the sterility of the water can be ensured by the other of the sterilizers.

As shown in Fig. 3B, the water sterilizer 50 may include a foreign matter removal filter 51, the first sterilizer 52, the first sterilizing filter 53, the second sterilizer 54, and the second sterilizing filter 55. The water sterilizer 50 may further include the pre-sterilizer 56 provided upstream of the foreign matter removal filter 51. The pre-sterilizer 56, the foreign matter removal filter 51, the first sterilizer 52, the first sterilizing filter 53, the second sterilizer 54, and the second sterilizing filter 55 are arranged in this order from the upstream side to the downstream side along the conveying direction of water. Since the water sterilizer 50 includes a plurality of sterilizing filters (the first sterilizing filter 53 and the second sterilizing filter 55), even when an abnormality occurs in one of the sterilizing filters, the sterility of the water can be ensured by the other of the sterilizing filters. The order in which the first sterilizer 52, the first sterilizing filter 53, the second sterilizer 54, and the second sterilizing filter 55 are arranged is not limited thereto. The number of sterilizing filters and sterilizers included in the water sterilizer 50 is also not limited thereto. For example, although not shown in the drawing, the water sterilizer 50 may include only the first sterilizer 52 or the second sterilizer 54, and the first sterilizing filter 53. Alternatively, two water sterilizers 50 may be provided in parallel.

Alternatively, the water sterilizer 50 does not need to include a sterilizing filter. In other words, depending on the sterile quality level (sterility assurance level (SAL)) of the contents prepared by diluting the product concentrate with water, when a microbial count level (concentration) of water supplied to the first water tank 41 is low, due to the growth characteristics of microorganisms in the contents, and/or the like, the water sterilizer 50 does not need to include a sterilizing filter in some cases. Also, when sterilized water is used for cleaning (cleaning out place (COP)) and/or sterilizing (sterilizing out place (SOP)) in each chamber, the water does not come into direct contact with the contents. In this case as well, there are cases where the water sterilizer 50 does not need to include a sterilizing filter. In this case, for example, although not shown in the drawing, the water sterilizer 50 may include only the first sterilizer 52 or may include the first sterilizer 52 and the second sterilizer 54. In this way, when the water sterilizer 50 does not include a sterilizing filter, the manufacturing cost of the water sterilizer 50 can be reduced.

Furthermore, the water sterilizer 50 does not need to include a sterilizer. In other words, depending on the sterile quality level of the contents prepared by diluting the product concentrate with water, when a microbial count level of water supplied to the first water tank 41 is low, due to the growth characteristics of microorganisms in the contents, and/or the like, the water sterilizer 50 does not need to include a sterilizer in some cases. For example, when the contents are a carbonated beverage containing alcohol having a pH of lower than 4.5, the water sterilizer 50 does not need to include a sterilizer. In this case, for example, although not shown in the drawing, the water sterilizer 50 may include only the first sterilizing filter 53 or may include the first sterilizing filter 53 and the second sterilizing filter 55. In this way, when the water sterilizer 50 does not include a sterilizer, the manufacturing cost of the water sterilizer 50 can be reduced.

Next, the pre-sterilizer 56, the foreign matter removal filter 51, the first sterilizer 52, the first sterilizing filter 53, the second sterilizer 54, and the second sterilizing filter 55 will be described. Here, first, the pre-sterilizer 56 will be described.

The pre-sterilizer 56 preliminarily sterilizes water supplied to the foreign matter removal filter 51. Since the pre-sterilizer 56 is installed upstream of the foreign matter removal filter 51, high-quality sterile water can be produced for a long period of time. The configuration of the pre-sterilizer 56 may have substantially the same configuration as the first sterilizer 52.

The foreign matter removal filter 51 removes foreign matter in water. In the illustrated example, the water sterilizer 50 includes the single foreign matter removal filter 51. However, the water sterilizer 50 is not limited to including the single foreign matter removal filter 51 and may include a plurality of foreign matter removal filters 51. The pore size (filtration precision) of the foreign matter removal filter 51 may be, for example, greater than or equal to 0.20 µm and less than or equal to 10 µm and may be greater than or equal to 0.45 µm and less than or equal to 5 µm. The pore size of the foreign matter removal filter 51 is preferably such that fungi (such as molds and yeasts) can be removed. As described later, ultraviolet light is irradiated onto the water in the first sterilizer 52 and the like provided downstream of the foreign matter removal filter 51. Therefore, the pore size of the foreign matter removal filter 51 is preferably such that molds resistant to ultraviolet light can be removed, and is preferably greater than or equal to 0.45 µm and less than or equal to 1.2 µm. In order to improve the sterility of water passing through the foreign matter removal filter 51, the pore size of the foreign matter removal filter 51 may be greater than or equal to 0.2 µm and less than or equal to 0.45 µm. When the pore size of the foreign matter removal filter 51 is less than or equal to 0.45 µm, substantially all microorganisms remaining in the water can be captured. In order to improve the sterility of water passing through the foreign matter removal filter 51, a sterile-grade filter having a pore size of greater than or equal to 0.1 µm and less than or equal to 0.22 µm may be used as the foreign matter removal filter 51. The filtration membrane of the foreign matter removal filter 51 may be, for example, a reverse osmosis (RO) membrane or an ultra-filtration (UF) membrane.

The first sterilizer 52 is provided downstream of the foreign matter removal filter 51. The first sterilizer 52 is provided upstream of the first sterilizing filter 53. The first sterilizer 52 is a sterilizer that sterilizes water with ultraviolet light. Ultraviolet light can sterilize microorganisms (bacteria other than molds and yeasts) that have passed through the foreign matter removal filter 51. Since the first sterilizer 52 sterilizes water with ultraviolet light, carbon dioxide emissions from the content filling system can be reduced as compared with when water is sterilized by heating. In particular, as described above, when contents are produced, the product concentrate can be diluted with water by a factor greater than or equal to 1.1 times and less than or equal to 1000 times, and preferably by a factor greater than or equal to 2 times and less than or equal to 10 times. When the product concentrate is diluted with water by a factor greater than or equal to 2 times and less than or equal to 10 times, 50% or higher and 90% or lower of the contents are water. Therefore, by sterilizing water without heating, carbon dioxide emissions during production of the contents can be significantly reduced.

As described above, in the present embodiment, the first sterilizer 52 sterilizes water with ultraviolet light. In this case, the first sterilizer 52 may include an ultraviolet lamp. The ultraviolet lamp may be a low-pressure mercury lamp, a medium-pressure mercury lamp, a high-pressure mercury lamp, or an UV-LED. The first sterilizer 52 may include a plurality of ultraviolet lamps having different wavelengths and/or outputs of ultraviolet light to be irradiated. As an example, the first sterilizer 52 may include a low-pressure mercury lamp (including a low-pressure high-output amalgam lamp) and a medium-pressure mercury lamp.

A low-pressure mercury lamp is a mercury lamp in which the mercury vapor pressure during operation is lower than 10 Pa. The low-pressure mercury lamp can efficiently emit ultraviolet light having a wavelength (253.7 nm) with a high sterilization effect. When the first sterilizer 52 includes a low-pressure mercury lamp, ultraviolet light can be efficiently emitted, so the sterilization effect in the first sterilizer 52 (and the second sterilizer 54) can be improved. A low-pressure mercury lamp may be an amalgam lamp (low-pressure high-output amalgam lamp) in which an amalgam, which is an alloy of mercury and another metal, is enclosed in a discharge tube.

A medium-pressure mercury lamp is a mercury lamp in which the mercury vapor pressure during operation is higher than or equal to 40 kPa. The wavelength of ultraviolet light emitted by the medium-pressure mercury lamp has a dominant wavelength of 365 nm and has peaks also at 254 nm, 302 nm, 313 nm, 405 nm, 436 nm, and the like. Generally, medium-pressure mercury lamps are high-output mercury lamps as compared with low-pressure mercury lamps. Therefore, when the first sterilizer 52 includes a medium-pressure mercury lamp, a large amount of water can be sterilized by the first sterilizer 52 (and the second sterilizer 54). Since the medium-pressure mercury lamp is a high-output mercury lamp, when a first ultraviolet lamp 67a and a second ultraviolet lamp 67b are both medium-pressure mercury lamps, the size of the first sterilizer 52 (and the second sterilizer 54) can be reduced.

Since a medium-pressure mercury lamp has higher heat resistance than a low-pressure mercury lamp, the medium-pressure mercury lamp can be operated at high temperatures. Therefore, in the circulation system 44A including the first sterilizer 52 and the second sterilizer 54, when the first sterilizer 52 and the second sterilizer 54 are sterilized by circulating heated water or a sterilizing agent, the first sterilizer 52 and the like can be sterilized in a state where the first ultraviolet lamp 67a and the like are turned on.

In the present embodiment, the cumulative irradiation dose of ultraviolet light applied to the water is preferably greater than or equal to 10 mJ/cm² and less than or equal to 10000 mJ/cm², and more preferably greater than or equal to 100 mJ/cm² and less than or equal to 1000 mJ/cm². In other words, when the water passes through the first sterilizer 52, the cumulative irradiation dose of ultraviolet light applied to the water is preferably greater than or equal to 10 mJ/cm² and less than or equal to 10000 mJ/cm², and more preferably greater than or equal to 100 mJ/cm² and less than or equal to 1000 mJ/cm². In this case, the cumulative irradiation dose of ultraviolet light applied to the water at a wavelength of 254 nm is preferably greater than or equal to 10 mJ/cm² and less than or equal to 10000 mJ/cm², and more preferably greater than or equal to 100 mJ/cm² and less than or equal to 1000 mJ/cm². When the cumulative irradiation dose of ultraviolet light is greater than or equal to 10 mJ/cm², aquatic microorganisms (gram-negative bacteria such as the Pseudomonas genus or the Methylobacterium genus that can proliferate in oligotrophic aquatic environments) that may pass through the second sterilizing filter 55 can be effectively sterilized. Also, since the cumulative irradiation dose of ultraviolet light is greater than or equal to 100 mJ/cm², bacterial spores can also be sterilized. When the cumulative irradiation dose of ultraviolet light is less than or equal to 10000 mJ/cm², electricity consumption can be reduced, and carbon dioxide emissions from the content filling system 10 can be reduced. Here, the wavelength of ultraviolet light may be longer than or equal to 250 nm and shorter than or equal to 260 nm, and may be, for example, 253.7 nm (254 nm). When the wavelength of ultraviolet light is longer than or equal to 250 nm and shorter than or equal to 260 nm, and is especially 253.7 nm, the sterilization effect of ultraviolet light on microorganisms can be enhanced. Here, in this specification, the term "aquatic microorganisms" means microorganisms (filterable bacteria) that can pass through a sterilizing filter having a pore size of 0.2 µm.

The first sterilizer 52 is preferably capable of being sterilized (sterilization in place (SIP)). Since the first sterilizer 52 is capable of being sterilized, the first sterilizer 52 can be periodically sterilized. When the first sterilizer 52 is sterilized, the first sterilizer 52 may be sterilized with vapor or heated water. Alternatively, when the first sterilizer 52 is heat-sensitive, the first sterilizer 52 may be sterilized by circulating a sterilizing agent containing, for example, peracetic acid in the circulation system 44A including the water sterilizer 50. When a sterilizing agent is circulated, the sterilizing agent may be circulated in the circulation system 44A for at least longer than or equal to 10 seconds and shorter than or equal to 60 minutes. Alternatively, by circulating a cleaning agent containing an acid or an alkali in the circulation system 44A including the water sterilizer 50, the first sterilizer 52 may be sterilized while being cleaned. In this case, in the circulation system 44A, a cleaning agent may be circulated for at least longer than or equal to 10 seconds and shorter than or equal to 60 minutes.

When a cleaning agent is circulated in the circulation system 44A, the temperature of the cleaning agent may be higher than or equal to 40°C and lower than or equal to 150°C and preferably higher than or equal to 50°C and lower than 100°C. When an acid is used as a cleaning agent, nitric acid, phosphoric acid, peracetic acid, acetic acid, hydrogen peroxide, pernitric acid, or the like may be used as the cleaning agent. An alkaline detergent may be a cleaning solution to which an alkaline agent is added. The alkaline agent is a mixture of caustic soda, potassium hydroxide, sodium carbonate, sodium silicate, sodium phosphate, sodium hypochlorite, a surfactant, a chelating agent, and the like. When a cleaning agent is circulated in the circulation system 44A, the cleaning agent may be circulated while the ultraviolet lamp is turned on. After cleaning, pure water is supplied to the circulation line 44 to perform rinsing until the cleaning agent is removed from the circulation line 44. When rinsing is performed until the cleaning agent is removed from the circulation line 44, the concentration of the cleaning agent in the circulation line 44 may be monitored with a concentration meter (not shown). After the circulation line 44 has been rinsed, an integrity test of the first sterilizing filter 53 and the like may be performed. Then, in the integrity test, when no leakage of sterile air from the filter is detected, the process may proceed to production. In a series of steps including the above-described cleaning and sterilization, rinsing, and integrity test of the water sterilizer 50, and production (from the start of production to the end of production), the water may be constantly circulated while the ultraviolet lamp is turned on. The ultraviolet lamp is turned on for a period from at least the sterilization step until the end of production. By turning on the ultraviolet lamp from the sterilization step onward, the flow of microorganisms that have passed through the filter to the second water tank 42 and downstream can be reduced.

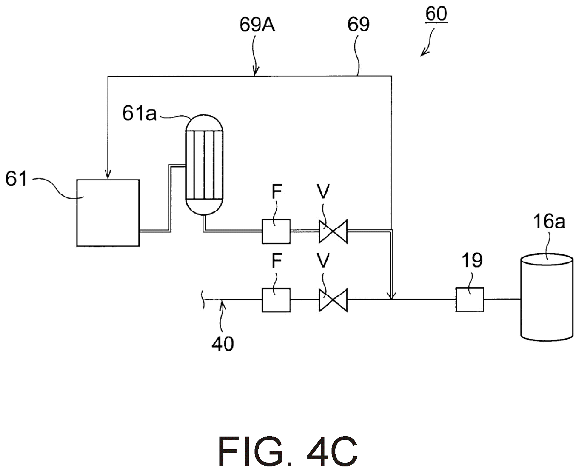

The first sterilizing filter 53 is provided downstream of the first sterilizer 52. The first sterilizing filter 53 is a micro-filtration (MF) filter that removes microorganisms from water by capturing microorganisms remaining in the water. The pore size of the first sterilizing filter 53 may be greater than or equal to 0.1 µm and less than or equal to 0.45 µm, and is preferably greater than or equal to 0.1 µm and less than or equal to 0.22 µm. When the pore size of the first sterilizing filter 53 is greater than or equal to 0.1 µm, a reduction in the sterilization efficiency of water can be suppressed. When the pore size of the first sterilizing filter 53 is less than or equal to 0.45 µm, microorganisms remaining in water can be effectively captured by the first sterilizing filter 53. A filter having a pore size of greater than or equal to 0.02 µm and less than or equal to 0.1 µm and capable of removing some viruses may be used as the first sterilizing filter 53. As described later, when the product concentrate is sterilized by filtration, the pore size of the first sterilizing filter 53 may be matched to the pore size of the filtration sterilizing filter 61a (see Fig. 4C) (described later) of the concentrate sterilization line 60. In this case, a difference between the pore size of the first sterilizing filter 53 and the pore size of the filtration sterilizing filter 61a may be, for example, greater than or equal to 0 µm and less than or equal to 0.8 µm. The material of the filtration membrane (membrane) of the first sterilizing filter 53 may be, for example, polyvinylidene fluoride (PVDF), polyether sulfone (PES), mixed cellulose (SCWP), polycarbonate (PC), polypropylene (PP), polyamide, or the like. The filtration membrane of the first sterilizing filter 53 may be, for example, a reverse osmosis (RO) membrane or an ultra-filtration (UF) membrane.

The first sterilizing filter 53 is preferably capable of being sterilized (SIP). Since the first sterilizing filter 53 is capable of being sterilized, the first sterilizing filter 53 can be periodically sterilized. Here, as described above, the first sterilizing filter 53 captures microorganisms that pass through the first sterilizer 52 and remain in the water. Therefore, when sterilization of water is continued for a long period of time in the water sterilizer 50, the captured microorganisms can proliferate in the first sterilizing filter 53. When dead microorganisms, which are organic matter, adhere to the first sterilizing filter 53 or the like, the dead microorganisms can serve as a substrate. In this case, microorganisms can further proliferate in the first sterilizing filter 53. In this way, when microorganisms proliferate in the first sterilizing filter 53, the microorganisms may enter water that passes through the first sterilizing filter 53. In contrast, when the first sterilizing filter 53 is capable of being sterilized, entry of microorganisms, adhering to the first sterilizing filter 53, into water that passes through the first sterilizing filter 53 can be suppressed. As a result, a decrease in the filtration performance of the first sterilizing filter 53 can be suppressed.

Preferably, an integrity test for the pore size of the first sterilizing filter 53 can be performed. Here, the integrity test may be, for example, performed by a bubble point test. The bubble point test can be performed as follows. For example, first, by supplying water to a housing (not shown) in the first sterilizing filter 53, the filter (not shown) of the first sterilizing filter 53 is covered with water. Subsequently, supply of water is stopped, and water in the first sterilizing filter 53 is drained. In this case, the water is drained using a blow line (not shown) while maintaining a sterile state in the first sterilizing filter 53. Then, sterile air is supplied from a primary side in the first sterilizing filter 53 in which the filter is covered with water. Subsequently, the supply pressure of sterile air is increased until the sterile air supplied from the primary side of the first sterilizing filter 53 passes through to the secondary side. Then, the pore size of the first sterilizing filter 53 is determined based on the pressure of sterile air at which the sterile air passes through the first sterilizing filter 53 (bubble point). Thus, since an integrity test for the pore size of the first sterilizing filter 53 can be performed, the degree of degradation of the first sterilizing filter 53 can be easily determined. The integrity test may be performed by a diffusion flow test, a pressure hold test, or the like, in addition to the above-described bubble point test.

The second sterilizer 54 is provided downstream of the first sterilizing filter 53. The configuration of the second sterilizer 54 may be substantially the same as the configuration of the first sterilizer 52. In other words, the second sterilizer 54 may sterilize water with ultraviolet light.

The second sterilizing filter 55 is provided downstream of the second sterilizer 54. The second sterilizing filter 55 removes microorganisms from water by capturing microorganisms that pass through the second sterilizer 54 and remain in the water. The pore size of the second sterilizing filter 55 may be equivalent to the pore size of the first sterilizing filter 53. In this case, two sterilization sets, each including a sterilizer and a sterilizing filter, can be arranged along the conveying direction of water. In other words, a first sterilization set including the first sterilizer 52 and the first sterilizing filter 53 and a second sterilization set including the second sterilizer 54 and the second sterilizing filter 55 can be arranged in series along the conveying direction of water. Therefore, even when some abnormality occurs in one of the sterilization sets, the sterility of the water can be ensured. The pore size of the second sterilizing filter 55 may be less than or equal to the pore size of the first sterilizing filter 53. As a result, even if microorganisms in the water pass through the first sterilizing filter 53, the microorganisms can be captured by the second sterilizing filter 55. Therefore, the sterility of the water can be sufficiently ensured. A plurality of sterilization sets may be provided in accordance with the sterility assurance level (SAL) of water or a final product (contents). Although not shown in the drawing, the number of sterilization sets may be one or may be three or more.

The pore size of the second sterilizing filter 55 may be greater than or equal to 0.1 µm and less than or equal to 0.45 µm, and is preferably greater than or equal to 0.1 µm and less than or equal to 0.22 µm. When the pore size of the second sterilizing filter 55 is greater than or equal to 0.1 µm, a reduction in the sterilization efficiency of water can be suppressed. When the pore size of the second sterilizing filter 55 is less than or equal to 0.45 µm, microorganisms remaining in water can be further effectively captured by the second sterilizing filter 55. As described later, when the product concentrate is sterilized by filtration, the pore size of the second sterilizing filter 55 may be matched to the pore size of the filtration sterilizing filter 61a (see Fig. 4C) (described later) of the concentrate sterilization line 60. In this case, a difference between the pore size of the second sterilizing filter 55 and the pore size of the filtration sterilizing filter 61a may be, for example, greater than or equal to 0 µm and less than or equal to 0.8 µm. The filtration membrane of the second sterilizing filter 55 may be, for example, a reverse osmosis (RO) membrane or an ultra-filtration (UF) membrane.

The remaining configuration of the second sterilizing filter 55 may be substantially the same as the configuration of the first sterilizing filter 53. In other words, the second sterilizing filter 55 may be capable of being sterilized (SIP). An integrity test for the pore size of the second sterilizing filter 55 may be able to be performed.

The processing capacity of the water sterilizer 50 is preferably 105% or higher of the maximum processing capacity needed during production of the product can 1A, and more preferably 110% or higher of the maximum processing capacity needed during production of the product can 1A. For example, the processing capacity of the water sterilizer 50 may be higher than or equal to 5 m³/h and lower than or equal to 50 m³/h and may be, for example, 24 m³/h. When the processing capacity of the water sterilizer 50 is 105% or higher of the maximum processing capacity needed during production of the product can 1A, a predetermined amount of water can be stored in the second water tank 42 during production of the product can 1A. In this case, by designing the volume of the second water tank 42 as needed, production of the product can 1A and sterilization and the like of the first sterilizing filter 53 and the like can be performed without shortage of water even during the above-described cleaning (CIP), sterilization (SIP), or integrity test of the first sterilizing filter 53 or the like, or during maintenance or the like of the first sterilizing filter 53 or the like. The time needed for sterilization (SIP) of the first sterilizing filter 53 or the like and the time needed for an integrity test each are longer than or equal to about 30 minutes and shorter than or equal to about one hour. Therefore, the volume of the second water tank 42 may be larger than or equal to the amount of water used in the content filling system 10 when the product can 1A is produced for one hour.

Here, as described above, the content filling system 10 includes the control unit 100 (see Fig. 1) that controls the content filling system 10. The control unit 100 may be electrically connected to the container sterilization apparatus 20, the lid sterilization apparatus 30, the water sterilization line 40, the concentrate sterilization line 60, the filling apparatus (filler) 70, and the lid attaching apparatus (seamer) 80, and the control unit 100 may control the water sterilization line 40 and the like. Then, the control unit 100 may clean the lid attaching apparatus 80 with water that has been sterilized without heating by the water sterilization line 40.

Next, the concentrate sterilization line 60 will be described. The concentrate sterilization line 60 is a sterilization line that sterilizes a product concentrate.

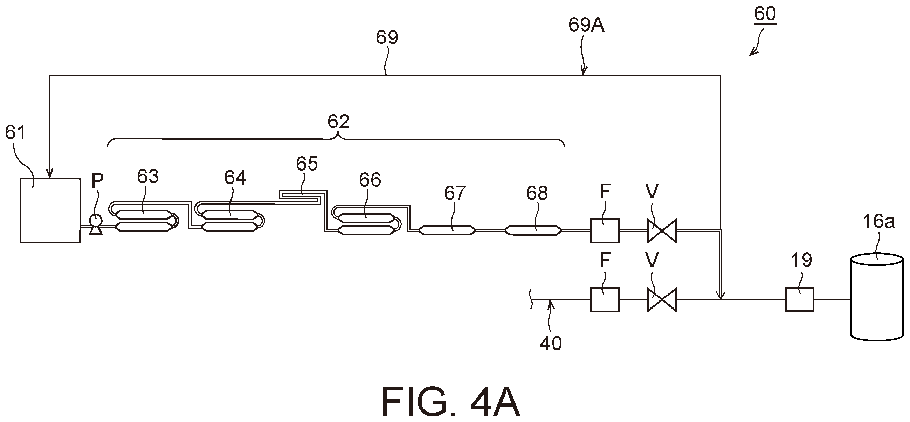

As shown in Fig. 4A, the concentrate sterilization line 60 includes a concentrate tank 61 and a product concentrate sterilizer 62. The concentrate tank 61 and the product concentrate sterilizer 62 are arranged in this order from the upstream side to the downstream side along the conveying direction of the product concentrate. In the concentrate sterilization line 60, a flowmeter F and a sterile valve V with an adjustable opening may be provided downstream of the product concentrate sterilizer 62. Although not shown in the drawing, thermometers, pressure gauges, level gauges, concentration meters, and the like are installed at various locations in the concentrate sterilization line 60.

The concentrate tank 61 stores a product concentrate supplied from a supply source (not shown). The concentrate tank 61 serves to smooth the flow of a product concentrate by storing the product concentrate. The volume of the concentrate tank 61 may be larger than or equal to 0.3 m³ and smaller than or equal to 30 m³ and may be, for example, 1 m³.

A pump P for conveying a product concentrate may be provided downstream of the concentrate tank 61. The above-described product concentrate sterilizer 62 may be provided downstream of the pump P.

The product concentrate sterilizer 62 is a sterilizer that sterilizes, by heating, a product concentrate stored in the concentrate tank 61. In the present embodiment, the product concentrate sterilizer 62 may be a sterilizer (ultra high-temperature; hereinafter, simply referred to as UHT) that sterilizes a product concentrate by an ultra-high temperature heat treatment method. The UHT 62 includes a first-stage heating unit 63, a second-stage heating unit 64, a holding tube 65, a first-stage cooling unit 66, a second-stage cooling unit 67, and a third-stage cooling unit 68. The product concentrate supplied to the UHT 62 is gradually heated by the first-stage heating unit 63 and the second-stage heating unit 64, and is heated to a target temperature in the holding tube 65. In this case, for example, a product concentrate may be heated to a temperature higher than or equal to 60°C and lower than or equal to 80°C by the first-stage heating unit 63, and then heated to a temperature higher than or equal to 80°C and lower than or equal to 150°C by the second-stage heating unit 64. The temperature of the product concentrate is maintained for a certain period of time in the holding tube 65. The product concentrate that has passed through the holding tube 65 is gradually cooled by the first-stage cooling unit 66, the second-stage cooling unit 67, and the third-stage cooling unit 68. The number of stages of the heating unit and the cooling unit is increased or decreased as needed. A pressure loss of the product concentrate can be high between the first-stage heating unit 63 and the second-stage heating unit 64. Therefore, an additional pump (not shown) may be provided between the first-stage heating unit 63 and the second-stage heating unit 64. A homogenizer for homogenizing the product concentrate may be provided between the first-stage heating unit 63 and the second-stage heating unit 64, or between the first-stage cooling unit 66 and the second-stage cooling unit 67.

The processing capacity of the UHT 62 may be higher than or equal to 3 m³/h and lower than or equal to 30 m³/h and may be, for example, 6 m³/h.

By monitoring the temperature of a location where the temperature is the highest (for example, the second-stage heating unit 64) within the UHT 62, a scale (deposit, such as calcium) adhering to the UHT 62 may be monitored. When the UHT 62 is cleaned (cleaning in place (CIP)), the removal state of the scale may be monitored. By monitoring, a cleaning step of cleaning the UHT 62 can be optimized. Therefore, the cleaning time can be shortened, and the usage of water, vapor, and cleaning agent used for cleaning can be reduced. As a result, carbon dioxide emissions from the content filling system 10 can be reduced.

The UHT 62 may be of an injection type or may be of an infusion type. A heat exchanger used to exchange heat in the content filling system 10, such as a heat exchanger of the UHT 62, may be of a plate type, may be of a shell-and-tube type, may be of a scraped-surface type, or may be of a Joule heating (ohmic heating) type. In the content filling system 10, these heat exchangers may be used in combination.

In the above-described example, an example in which the concentrate sterilization line 60 sterilizes a product concentrate by heating has been described; however, the configuration is not limited thereto. The concentrate sterilization line 60 may sterilize a product concentrate by at least one of heating, storage, and filtration. For example, the concentrate sterilization line 60 may sterilize a product concentrate by storing the product concentrate in the concentrate tank 61. In this case, the product concentrate may contain alcohol. By containing alcohol, the sterilization treatment of the product concentrate can be performed using the sterilization effect of alcohol. When the product concentrate is sterilized, the product concentrate can be stored in the concentrate tank 61 for a certain period of time. The storage time of the product concentrate significantly depends on the alcohol concentration. When the alcohol concentration is high, the storage time may be longer than or equal to 10 minutes. When the storage time is longer than or equal to 10 minutes, the sterilization effect on the product concentrate can be improved. When the alcohol concentration is low, the storage time may be shorter than or equal to 24 hours. When the storage time is shorter than or equal to 24 hours, the sterilization time of the product concentrate can be shortened.

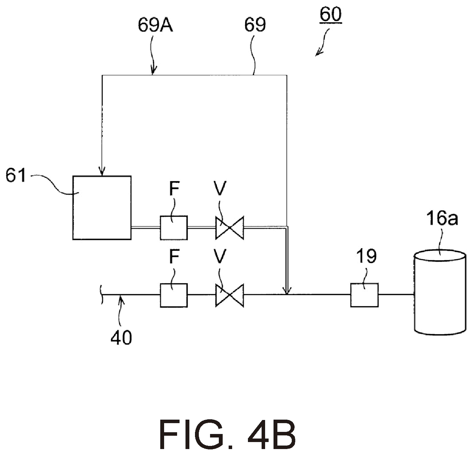

As shown in Fig. 4B, when the concentrate sterilization line 60 sterilizes a product concentrate by storage, the concentrate sterilization line 60 does not need to include the product concentrate sterilizer 62. A product concentrate may be sterilized using another tank (not shown) different from the concentrate tank 61. Furthermore, when the product concentrate is sterilized, sterilization treatment may be performed by batch processing using a plurality of tanks. Alternatively, when a product concentrate is sterilized, sterilization treatment may be performed by continuously supplying the product concentrate to the concentrate tank 61 or the like while storing the product concentrate in the concentrate tank 61 or the like for a certain period of time.

When there are no quality issues after the product concentrate is sterilized by filtration, filtration sterilization may be adopted as a sterilization method for the product concentrate. In this case, as shown in Fig. 4C, the concentrate sterilization line 60 may include the concentrate tank 61 and the filtration sterilizing filter 61a. The concentrate tank 61 and the filtration sterilizing filter 61a are arranged in this order from the upstream side to the downstream side along the conveying direction of the product concentrate. The filtration sterilizing filter 61a may have the same configuration as the above-described first sterilizing filter 53 or second sterilizing filter 55. In the concentrate sterilization line 60, a flowmeter F and a sterile valve V with an adjustable opening may be provided downstream of the filtration sterilizing filter 61a. Although not shown in the drawing, the concentrate sterilization line 60 may also include the product concentrate sterilizer 62.

As described above, the concentrate sterilization line 60 may sterilize a product concentrate by at least one of heating, storage, and filtration. For example, the concentrate sterilization line 60 may sterilize the product concentrate by heating and storage, or may sterilize the product concentrate by heating, storage, and filtration. The concentrate sterilization line 60 may sterilize the product concentrate by ultra-high pressure sterilization or another sterilization method.

As shown in Figs. 4A to 4C, a circulation line 69 may be connected to the concentrate sterilization line 60. One end of the circulation line 69 may be connected to the downstream side of the sterile valve V with an adjustable opening, and the other end of the circulation line 69 may be connected to the concentrate tank 61. As a result, a circulation system 69A that circulates a product concentrate may be formed. In the example shown in Fig. 4A, the circulation system 69A is constituted by the concentrate tank 61, the product concentrate sterilizer 62, and the circulation line 69. In the example shown in Fig. 4B, the circulation system 69A is constituted by the concentrate tank 61 and the circulation line 69. In the example shown in Fig. 4C, the circulation system 69A is constituted by the concentrate tank 61, the filtration sterilizing filter 61a, and the circulation line 69. A thermometer (not shown) may be provided on the circulation line 69. A concentration meter (not shown) for measuring the concentration of a sterilizing agent or a cleaning agent when the product concentrate sterilizer 62 is sterilized may be provided on the circulation line 69. Furthermore, a heating device (such as a heat exchanger and a heater (not shown)) for heating a sterilizing agent or the like during cleaning and/or sterilization of the circulation line 69 may be installed on the circulation line 69.

Here, the pipe of the concentrate sterilization line 60 is connected to the pipe of the water sterilization line 40. Then, a product concentrate may be mixed into flowing water at a constant ratio. The contents can be blended by diluting (mixing) a product concentrate with water before filling. When blending is performed, a sterile valve V with an adjustable opening may be provided at a downstream end of the water sterilization line 40 and a sterile valve V with an adjustable opening may be provided at a downstream end of the concentrate sterilization line 60 in order to make the flow rate of water from the water sterilization line 40 and the flow rate of the product concentrate from the concentrate sterilization line 60 adjustable. Then, the opening and closing of each sterile valve V may be adjusted such that the dilution ratio of the product concentrate to water becomes a predetermined ratio. The pipe of the concentrate sterilization line 60 may be connected perpendicularly to the pipe of the water sterilization line 40. The pipe of the water sterilization line 40 and the pipe from the intersection of the pipe of the water sterilization line 40 with the pipe of the concentrate sterilization line 60 to a storage tank 16a (described later) may be arranged in a straight line. In the pipe from the intersection of the pipe of the water sterilization line 40 with the pipe of the concentrate sterilization line 60 to the storage tank 16a (described later), a positive pressure is held by the water supplied from the water sterilization line 40 and the product concentrate supplied from the concentrate sterilization line 60. Even when water and/or a product concentrate is drained, since the pipe from the above-described intersection to the storage tank 16a (described later) is connected to the storage tank 16a or the second water tank 42, a positive pressure is held inside the pipe. An agitator (not shown) is installed in the storage tank 16a.

Next, the base alcohol line 110 will be described. The base alcohol line 110 adds alcohol to the sterilized water and the sterilized product concentrate.

As shown in Fig. 1, the base alcohol line 110 is connected between the intersection of the pipe of the water sterilization line 40 with the pipe of the concentrate sterilization line 60 and a static mixer 19 (described later). The base alcohol line 110 includes a base alcohol tank 111 that stores base alcohol. A flowmeter F and a sterile valve V with an adjustable opening may be provided downstream of the base alcohol tank 111. Although not shown in the drawing, thermometers, pressure gauges, level gauges, concentration meters, pumps, and the like are installed at various locations in the base alcohol line 110.

The base alcohol tank 111 stores base alcohol supplied from a supply source (not shown). The base alcohol tank 111 serves to smooth the flow of base alcohol by storing the base alcohol. The volume of the base alcohol tank 111 may be larger than or equal to 0.3 m³ and smaller than or equal to 30 m³ and may be, for example, 1 m³.

The static mixer 19 may be provided downstream of the intersection of the pipe of the water sterilization line 40 with the pipe of the concentrate sterilization line 60. Then, the two liquids (water and the product concentrate) may be further homogenized by the static mixer 19.

The storage tank (so-called aseptic tank) 16a that stores blended contents may be provided downstream of the static mixer 19. A concentration meter that measures the concentration of the blended contents may be installed in the storage tank 16a. The volume of the storage tank 16a may be larger than or equal to 0.1 m³ and smaller than or equal to 30 m³ and may be, for example, 0.3 m³. Although not shown in the drawing, the pipe of the water sterilization line 40 and the pipe of the concentrate sterilization line 60 may be configured so as to independently supply water and the product concentrate from the water sterilization line 40 and the concentrate sterilization line 60 into the storage tank 16a without being connected to each other (tank blending).

As shown in Fig. 1, a cooling device 17 that cools contents may be provided downstream of the storage tank 16a. Furthermore, a carbonation device 18 that adds carbon dioxide to the cooled contents may be connected downstream of the cooling device 17. The carbonation device 18 may be a so-called aseptic carbonator.

Furthermore, a carbonated product tank 16b that stores contents to which carbon dioxide has been added may be provided downstream of the carbonation device 18. A so-called filler tank 16c may be provided downstream of the carbonated product tank 16b. The filler tank 16c is installed vertically above the filling apparatus 70 to improve the filling accuracy of the filling apparatus 70. Furthermore, the filler tank 16c may serve as a so-called cushion tank that ensures a smooth flow of contents even when the consumption of contents downstream of the filler tank 16c varies.

Carbon dioxide gas is supplied to the contents to maintain the carbonated product tank 16b and the filler tank 16c at a positive pressure. Carbon dioxide gas may be supplied to the contents after being filtered through a sterilizing filter having a pore size of greater than or equal to 0.2 µm and less than or equal to 0.45 µm. The sterilizing filter that filters carbon dioxide gas is sterilized by sterilization (SIP) before production. A sterilizing filter with a small pore size has low heat resistance, so, when the number of times sterilization is performed increases, it is difficult to use the sterilizing filter for a long period of time, and the sterilizing filter can be replaced in a short time. On the other hand, filtrable microorganisms have been reported to be present in water, whereas filtrable microorganisms are considered not to be present in air. Therefore, the pore size of the sterilizing filter used to filter carbon dioxide gas may be greater than or equal to the pore size of the sterilizing filter used to filter water. In other words, when carbon dioxide gas is filtered using a sterilizing filter, a filter having a pore size greater than or equal to the pore size of the filter used to sterilize water or a product concentrate by filtration may be used. Thus, the cost of the sterilizing filter for carbon dioxide gas can be reduced.

A concentration meter that measures the concentration of added carbon dioxide may be installed between the filler tank 16c and the carbonation device 18. The volume of the filler tank 16c may be larger than or equal to 0.1 m³ and smaller than or equal to 1 m³ and may be, for example, 0.3 m³.

Next, the filling apparatus 70 will be described. The filling apparatus 70 is an apparatus (filler) that fills contents subjected to sterilization treatment in advance into the can 1. In the filling apparatus 70, contents are filled into an empty can 1. The filling apparatus 70 may be a so-called rotary filler. In this case, in the filling apparatus 70, the contents are filled into a plurality of the cans 1 while the cans 1 are turned (revolved). The contents may be filled into the can 1 at room temperature. The contents are subjected to sterilization treatment in advance as described above and cooled to room temperature higher than or equal to 3°C and lower than or equal to 40°C, and are then filled into the can 1. Examples of the contents to be filled by the filling apparatus 70 include alcoholic beverages (low-alcoholic beverages) such as chuhai beverages, cocktail beverages, and liqueurs, and beer-based beverages and beer-taste beverages.

The can 1 filled with contents is sealed with a lid by the lid attaching apparatus 80.

The lid attaching apparatus 80 is an apparatus (seamer) that seals the can 1 by attaching the lid 2 to the can 1. In the lid attaching apparatus 80, the can 1 filled with water and a product concentrate (contents) is closed with the lid 2 and sealed such that external air and microorganisms do not enter the can 1. In the lid attaching apparatus 80, the lid 2 is attached (seamed) to the flange 1a. In this way, by attaching the lid 2 to the can 1, the product can 1A is obtained.

The lid 2 may be a lid made of aluminum. When the lid 2 is made of aluminum, the weight of the lid 2 can be reduced.

The weight of the lid 2 may be less than or equal to 4.0 g (206 end), may be less than or equal to 3.2 g (204 end), may be less than or equal to 2.5 g (202 end), and may be less than or equal to 2.0 g. When the lid 2 is made of the same material, the manufacturing cost of the lid 2 can be reduced as the weight of the lid 2 decreases. On the other hand, as the weight of the lid decreases, the lid is more susceptible to deformation. For this reason, when the weight of the lid is reduced, the lid may deform due to a pressure change during heat sterilization or the like. According to the present embodiment, since sterilization of the contents is performed before filling, a sterilization time after filling can be shortened or sterilization after filling can be omitted. Therefore, by using the content filling system 10 according to the present embodiment, deformation can be suppressed even when the weight of the lid 2 is less than or equal to 4.0 g, less than or equal to 3.2 g, or less than or equal to 2.5 g. The lid attaching apparatus 80 is not limited to a seamer. For example, when a resealable bottle can or the like is used as the can 1, a screw cap is used as the lid 2. In this case, a servo capper capable of torque control may be adopted as the lid attaching apparatus 80. Alternatively, depending on the type of the lid 2, a capping machine or the like may be adopted as the lid attaching apparatus 80.

The lid 2 is sterilized in advance by the lid sterilization apparatus 30. In the present embodiment, the lid sterilization apparatus 30 sterilizes the lid 2 by spraying a sterilizing agent onto the lid 2. Thus, the lid 2 is sterilized by the sterilizing agent before being attached to the can 1. The lid sterilization apparatus 30 is arranged, for example, near the lid attaching apparatus 80. In the lid sterilization apparatus 30, lids 2 carried in from outside the content filling system 10 are collected in advance in large numbers and conveyed in a line toward the lid attaching apparatus 80. While the lid 2 is being conveyed to the lid attaching apparatus 80, hydrogen peroxide gas or mist is sprayed toward the inner and outer surfaces of the lid 2, and then dried with hot air for sterilization treatment.

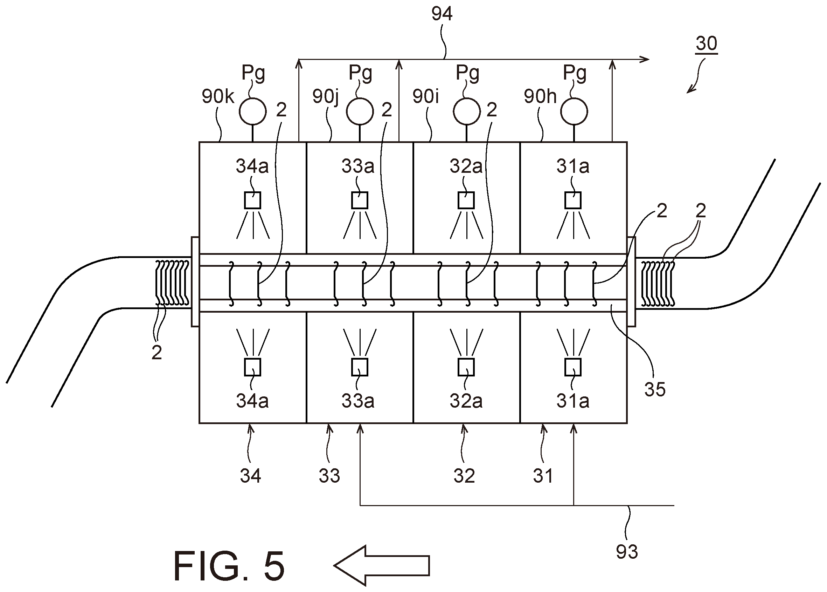

As shown in Figs. 1 and 5, the lid sterilization apparatus 30 includes a second sterilizing agent spraying unit 32 that sprays a sterilizing agent onto the lid 2, and a second air rinsing unit 33 that air-rinses the lid 2 onto which a sterilizing agent has been sprayed by the second sterilizing agent spraying unit 32. The lid sterilization apparatus 30 may further include a second heating unit 31 that is provided upstream of the second sterilizing agent spraying unit 32 and heats the lid 2. Furthermore, the lid sterilization apparatus 30 may further include a second sterile water rinsing unit 34 provided downstream of the second air rinsing unit 33. The second heating unit 31, the second sterilizing agent spraying unit 32, the second air rinsing unit 33, and the second sterile water rinsing unit 34 are arranged in this order from the upstream side to the downstream side along the conveying direction of the lid 2. As shown in Fig. 5, the lid 2 is conveyed in the direction of the arrow.

The second heating unit 31 is a portion that heats (preheats) the lid 2 before a sterilizing agent is sprayed. The second heating unit 31 is configured to heat the lid 2 while conveying the lid 2. When the lid 2 is heated while being conveyed, the lid 2 may be conveyed by a guide 35, such as a so-called screw chute, such that a spacing is provided between adjacent lids 2, as shown in Fig. 5. The second heating unit 31 includes second hot air nozzles 31a that heat the lid 2 by blowing heated air onto the lid 2. By heated air blown from the second hot air nozzles 31a, the lid 2 may be, for example, heated such that the surface has a temperature higher than or equal to 40°C and lower than or equal to 100°C or may be heated such that the surface has a temperature higher than or equal to 50°C and lower than or equal to 80°C. When the surface temperature of the lid 2 is higher than or equal to 40°C, the sterilization effect on the lid 2 can be improved. When the surface temperature of the lid 2 is lower than or equal to 100°C, thermal energy consumption can be suppressed, and carbon dioxide emissions can be reduced.

The second sterilizing agent spraying unit 32 is a portion that sterilizes the lid 2 by spraying a sterilizing agent onto the lid 2 heated by the second heating unit 31. The second sterilizing agent spraying unit 32 is configured to spray a sterilizing agent while conveying the lid 2. When a sterilizing agent is sprayed onto the lid 2 while the lid 2 is being conveyed, the lid 2 may be conveyed by the guide 35, such as a so-called screw chute, such that a spacing is provided between adjacent lids 2. A spacing may be provided between adjacent lids 2 by vibrating the guide 35. Then, a sterilizing agent may be applied to the lid 2 in a state where a spacing is provided between adjacent lids 2. The second sterilizing agent spraying unit 32 includes second spraying nozzles 32a that spray a sterilizing agent onto the lid 2 being conveyed. At each second spraying nozzle 32a, the discharge pressure of sterilizing agent may be higher than or equal to 0.4 MPa. A plurality of the second spraying nozzles 32a may be used to apply a sterilizing agent to the lid 2. For example, a hydrogen peroxide aqueous solution is used as the sterilizing agent sprayed by the second sterilizing agent spraying unit 32. In the second sterilizing agent spraying unit 32, a gas or a mist of the hydrogen peroxide aqueous solution is generated, and the gas or the mist is sprayed from the second spraying nozzles 32a onto the inner and outer surfaces of the lid 2. In this way, since the lid 2 is sterilized with a gas or a mist of hydrogen peroxide aqueous solution, the inner and outer surfaces of the lid 2 are uniformly sterilized.

In the second sterilizing agent spraying unit 32, when the inner surface of the lid 2 is coated with at least one of epoxy resin, PET resin, and polyvinyl chloride, the amount of sterilizing agent adhering to the lid 2 may be greater than or equal to 0.01 µL/cm² and less than or equal to 0.4 µL/cm² and may be greater than or equal to 0.03 µL/cm² and less than or equal to 0.1 µL/cm². When the amount of sterilizing agent adhering to the lid 2 is greater than or equal to 0.01 µL/cm², the sterilization effect on the lid 2 can be improved. When the amount of sterilizing agent adhering to the lid 2 is greater than or equal to 0.03 µL/cm², the sterilization effect on the lid 2 can be further improved. When the amount of sterilizing agent adhering to the lid 2 is less than or equal to 0.4 µL/cm², a residue of the sterilizing agent on the lid 2 after passing through the lid sterilization apparatus 30 can be suppressed. In this case, the concentration of hydrogen peroxide in the sterilizing agent may be 35 wt%.

The second air rinsing unit 33 is a portion that supplies sterile heated air or room-temperature air to the lid 2 onto which a sterilizing agent has been sprayed in the second sterilizing agent spraying unit 32. When sterile heated air or room-temperature air is supplied, hydrogen peroxide is activated, and foreign matter, hydrogen peroxide, and the like, are removed from the inside of the lid 2. The second air rinsing unit 33 is configured to supply sterile air while conveying the lid 2. When sterile air is supplied onto the lid 2 while the lid 2 is being conveyed, the lid 2 may be conveyed by the guide 35, such as a so-called screw chute, such that a spacing is provided between adjacent lids 2. Thus, foreign matter can be effectively removed from the inside of the lid 2. If necessary, hydrogen peroxide may be gasified by mixing a low-concentration condensed mist of hydrogen peroxide with sterile air at room temperature, and supplied to the lid 2. Sterile air may be air, or may be carbon dioxide or an inert gas.

The second air rinsing unit 33 includes second air rinsing nozzles 33a that blow sterile air onto the lid 2 being conveyed. The second air rinsing nozzles 33a may blow sterile air onto the inner and outer surfaces of the lid 2. By blowing sterile air onto the inner and outer surfaces of the lid 2, a gas or a mist of hydrogen peroxide aqueous solution sprayed onto the inner and outer surfaces of the lid 2 is uniformly activated.

In the second air rinsing unit 33, sterile air at a temperature higher than or equal to 70°C and lower than or equal to 200°C may be blown onto the lid 2. For example, the temperature of the sterile air to be blown may be higher than or equal to 80°C, may be higher than or equal to 90°C, may be higher than or equal to 120°C, and may be higher than or equal to 150°C. When the temperature of the sterile air is higher than or equal to 70°C, a sterilizing agent adhering to the lid 2 can be effectively activated. When the temperature of the sterile air is lower than or equal to 200°C, carbon dioxide emissions can be reduced. The temperature of the sterile air is a temperature immediately after the sterile air is blown from the second air rinsing nozzles 33a, that is, a temperature at the tips of the second air rinsing nozzles 33a.

After sterile air is blown, the amount of sterilizing agent adhering to the lid 2 may be greater than or equal to 0.00001 µL/cm² and less than or equal to 0.01 µL/cm² when the inner surface of the lid 2 is coated with at least one of epoxy resin, PET resin, and polyvinyl chloride. When the amount of sterilizing agent adhering to the lid 2 is greater than or equal to 0.00001 µL/cm², the sterilization effect on the lid 2 can be improved. When the amount of sterilizing agent adhering to the lid 2 is less than or equal to 0.01 µL/cm², a residue of the sterilizing agent on the lid 2 after passing through the lid sterilization apparatus 30 can be suppressed.

The second sterile water rinsing unit 34 is a portion that cleans, with sterile water, the lid 2 sterilized by a sterilizing agent (hydrogen peroxide). By cleaning the lid 2 with sterile water, an extremely small amount of hydrogen peroxide adhering to the lid 2 is washed away, and foreign matter is removed. The second sterile water rinsing unit 34 is configured to supply sterile water while conveying the lid 2. When sterile water is supplied onto the lid 2 while the lid 2 is being conveyed, the lid 2 may be conveyed by the guide 35, such as a so-called screw chute, such that a spacing is provided between adjacent lids 2. Thus, the sterilizing agent and foreign matter can be effectively removed from the inside of the lid 2. In the second sterile water rinsing unit 34, the temperature of sterile water may be higher than or equal to 5°C and lower than or equal to 100°C.

As described above, the sterilized lid 2 is attached to the flange 1a of the can 1 at the lid attaching apparatus 80. In this way, the product can 1A is obtained. The obtained product can 1A is successively discharged toward the outside of the content filling system 10 by the product can discharge unit 11 (see Fig. 1).

As shown in Fig. 1, the product can discharge unit 11 may include a can warmer 12 that warms the product can 1A. The can warmer 12 may warm the product can 1A at about 5°C to about 30°C. By warming the product can 1A, occurrence of condensation on the surface of the product can 1A can be suppressed when the product can 1A is packaged. Therefore, for example, wetting of packaging material, such as cardboard, due to condensation can be suppressed.

The content filling system 10 includes a first heating chamber 90a, a first sterilizing agent spraying chamber 90b, a first air rinsing chamber 90c, a first sterile water rinsing chamber 90d, a filling chamber 90e, a lid attaching chamber 90f, and an outlet chamber 90g. The first heating chamber 90a, the first sterilizing agent spraying chamber 90b, the first air rinsing chamber 90c, the first sterile water rinsing chamber 90d, the filling chamber 90e, the lid attaching chamber 90f, and the outlet chamber 90g are arranged in this order from the upstream side to the downstream side along the conveying direction of the can 1.

The content filling system 10 includes a second heating chamber 90h, a second sterilizing agent spraying chamber 90i, a second air rinsing chamber 90j, and a second sterile water rinsing chamber 90k. The second heating chamber 90h, the second sterilizing agent spraying chamber 90i, the second air rinsing chamber 90j, the second sterile water rinsing chamber 90k, the lid attaching chamber 90f, and the outlet chamber 90g are arranged in this order from the upstream side to the downstream side along the conveying direction of the lid 2.

The chambers 90a to 90k are separated from one another by partition walls. The partition walls serve to suppress the flow of sterilizing agent and the like in unintended directions between the chambers 90a to 90k and to stabilize the pressure within each of the chambers 90a to 90k. Each partition wall has a spacing to such an extent that the can 1 or the lid 2 can pass through. The spacing is formed to have a minimum size, for example, about the size of one can 1 or one lid 2, so as to maintain the pressure within each of the chambers 90a to 90k. Each of the partition walls may include a shutter that closes the above-described spacing. The shutter may be, for example, configured to automatically open and close in response to a signal from the control unit 100. Furthermore, each of the chambers 90a to 90k is provided with a pressure gauge Pg (see, for example, Figs. 2 and 5).

As shown in Fig. 2, among the chambers 90a to 90k, the first heating chamber 90a houses the first heating unit 21 (first hot air nozzles 21a and 21b). The first sterilizing agent spraying chamber 90b houses the first sterilizing agent spraying unit 22 (first spraying nozzles 22a and 22b). The first air rinsing chamber 90c houses the first air rinsing unit 23 (first air rinsing nozzles 23a and 23b). Furthermore, the first sterile water rinsing chamber 90d houses the first sterile water rinsing unit 24 (first sterile water rinsing nozzles 24a and 24b). As shown in Fig. 2, a first sterile air supply line 91 for supplying sterile air is connected to the first heating chamber 90a and the first air rinsing chamber 90c. A first exhaust line 92 that exhausts air inside the chambers 90a, 90c, and 90d is connected to the first heating chamber 90a, the first air rinsing chamber 90c, and the first sterile water rinsing chamber 90d. A scrubber (not shown) that treats exhausted air is connected to the first exhaust line 92. On the other hand, in the illustrated example, the first exhaust line 92 is not connected to the first sterilizing agent spraying chamber 90b. Thus, a decrease in the gas concentration of the sterilizing agent in the first sterilizing agent spraying chamber 90b can be suppressed. When the pressure relationship (described later) within the chambers 90a to 90e can be maintained, the first exhaust line 92 may be connected to the first sterilizing agent spraying chamber 90b.

Referring again to Fig. 1, the filling chamber 90e houses the filling apparatus 70. The lid attaching chamber 90f houses the lid attaching apparatus 80. Furthermore, the outlet chamber 90g houses a conveyor (not shown) of the product can discharge unit 11.

As shown in Fig. 5, the second heating chamber 90h houses the second heating unit 31 (second hot air nozzles 31a). The second sterilizing agent spraying chamber 90i houses the second sterilizing agent spraying unit 32 (second spraying nozzles 32a). The second air rinsing chamber 90j houses the second air rinsing unit 33 (second air rinsing nozzles 33a). Furthermore, the second sterile water rinsing chamber 90k houses the second sterile water rinsing unit 34 (second sterile water rinsing nozzles 34a). As shown in Fig. 5, a second sterile air supply line 93 for supplying sterile air is connected to the second heating chamber 90h and the second air rinsing chamber 90j. A second exhaust line 94 that exhausts air inside the chambers 90h, 90j, and 90k is connected to the second heating chamber 90h, the second air rinsing chamber 90j, and the second sterile water rinsing chamber 90k. A scrubber (not shown) that treats exhausted air is connected to the second exhaust line 94. On the other hand, in the illustrated example, the second exhaust line 94 is not connected to the second sterilizing agent spraying chamber 90i. Thus, a decrease in the gas concentration of the sterilizing agent in the second sterilizing agent spraying chamber 90i can be suppressed. When the pressure relationship (described later) within the chambers 90e, 90f, and 90h to 90k can be maintained, the second exhaust line 94 may be connected to the second sterilizing agent spraying chamber 90i.

Next, the relationship among the pressures PA to PE within the chambers 90a to 90e will be described. The pressure PA in the first heating chamber 90a, the pressure PB in the first sterilizing agent spraying chamber 90b, the pressure PC in the first air rinsing chamber 90c, the pressure PD in the first sterile water rinsing chamber 90d, and the pressure PE in the filling chamber 90e may satisfy the following relationship. $0 \left(Pa\right) \leq PA \leq PB < PC \leq PD < PE ,$ or $PB \leq PA < PC \leq PD < PE$

In this case, the pressure PE in the filling chamber 90e is higher than the pressure PD in the first sterile water rinsing chamber 90d. As a result, entry of air in the first sterile water rinsing chamber 90d into the filling chamber 90e can be suppressed. Therefore, a sterile state inside the filling chamber 90e can be favorably maintained.

Next, the relationship among the pressures PE, PF, and PH to PK within the chambers 90e, 90f, and 90h to 90k will be described. The pressure PE in the filling chamber 90e, the pressure PF in the lid attaching chamber 90f, the pressure PH in the second heating chamber 90h, the pressure PI in the second sterilizing agent spraying chamber 90i, the pressure PJ in the second air rinsing chamber 90j, and the pressure PK in the second sterile water rinsing chamber 90k may satisfy the following relationship. $0 \left(Pa\right) \leq PH \leq PI < PJ \leq PK \leq PF < PE ,$ or $PI \leq PH < PJ \leq PK \leq PF < PE$

In this case, the pressure PE in the filling chamber 90e is higher than the pressure PF in the lid attaching chamber 90f. As a result, entry of air in the lid attaching chamber 90f into the filling chamber 90e can be suppressed. Therefore, a sterile state inside the filling chamber 90e can be favorably maintained. In this case, the pressure PF in the lid attaching chamber 90f is higher than or equal to the pressure PK in the second sterile water rinsing chamber 90k. As a result, entry of air in the second sterile water rinsing chamber 90k into the lid attaching chamber 90f can be suppressed. Therefore, a sterile state inside the lid attaching chamber 90f can be favorably maintained.

As shown in Fig. 1, each of the chambers 90c to 90g, 90j, and 90k may be provided with a sterile air supply device 95. The sterile air supply device 95 may include a blower and a sterilizing filter. Air that has passed through the blower of the sterile air supply device 95 is sterilized by the sterilizing filter. After that, the sterilized air becomes sterile air and is blown into the chambers 90c to 90g, 90j, and 90k. As the sterilizing filter, a high efficiency particulate air filter (HEPA filter) or an ultra low penetration air filter (ULPA filter) may be used.

Contents to be filled by the content filling system 10 may be an acidic carbonated beverage containing carbon dioxide gas. The beverage may be a ready-to-drink (RTD) beverage. Examples of the RTD beverage include beverages obtained by diluting brewed liquor, distilled liquor, or the like mainly containing alcohol (ethyl alcohol) with carbonated water. Specifically, alcoholic beverages (low-alcoholic beverages or alcohol-free beverages) such as chuhai beverages, cocktail beverages, wine-taste beverages, and liqueurs, or beer-based beverages (hereinafter, simply referred to as beer-based beverages and the like) can be filled into the can 1. The beverage filled in the can 1 may be a beverage with a high alcohol content. When the beverage has a high alcohol content, a sterilization step of sterilizing the product can 1A containing an alcoholic beverage may be omitted.

The Brix (sugar content) of the contents may be higher than or equal to 0% and lower than or equal to 30%, and may be higher than or equal to 5% and lower than or equal to 10%. When the Brix is lower than or equal to 30%, excessive viscosity of the contents can be suppressed. When the Brix is lower than or equal to 30%, flavor attributes, such as ripeness, fruitiness, and freshness can be effectively enhanced, while drinkability can be improved. Brix is a value obtained by converting a refractive index measured at 20°C into the mass/mass percentage of a sucrose solution based on a conversion table of International Commission for Uniform Methods of Sugar Analysis (ICUMSA) (unit: "°Bx", "%", or "degree"). Brix can be measured using a refractometer or the like.

Contents may contain fruit juices such that the sugar content falls within the above range. Examples of the fruit juices include lemon, orange, grapefruit, peach, strawberry, grape, lime, watermelon, melon, yuzu, shikuwasa (citrus depressa), and kumquat. The contents may contain two or more fruit juices.

For example, a concentrated fruit juice concentrate having a Brix of 50% is sterilized by heating at 100°C or higher for one minute, preferably at 110°C or higher for one minute or longer, and is then aseptically blended with water sterilized without heating. The dilution ratio is set to five-fold to achieve a Brix of 10%. The diluted contents are cooled to lower than or equal to 5°C. Carbon dioxide gas is added to the contents by an aseptic carbonator. In the aseptic carbonator, carbon dioxide gas is dissolved into the contents using carbon dioxide gas filtered by a 0.22 µm sterilizing air filter. After that, the contents are received in a carbonated liquid cushion tank and are filled under an aseptic environment. When the contents contain fruit juice and have a pH of lower than 4.5, thermo-acidophilic bacilli (TAB) serve as the indicator microorganisms (hazardous microorganisms) for heat sterilization. TAB are generally inactivated at about 110°C for about one minute. Here, when alcohol and carbon dioxide are added to the beverage, growth of TAB is suppressed, and thermo-resistant yeast serves as the indicator microorganism (hazardous microorganism). In the case of thermo-resistant yeast, since the thermo-resistant yeast is completely inactivated at about 90°C for about one minute, the temperature of a UHT (or a high temperature short time (HTST) sterilizer) can be lowered. As a result, microorganisms can be sterilized without impairing the color and flavor of the contents while the degradation of fruit juice components is suppressed.

When an acidic carbonated beverage containing carbon dioxide gas is filled into the can 1 as contents, the product can 1A may be a so-called high-gas product. A high-gas product is a product having a gas volume of greater than or equal to 3.0. In the present embodiment, the gas volume of the product can 1A may be greater than or equal to 3.0, and may be greater than or equal to 3.4.

### (Content Filling Method)

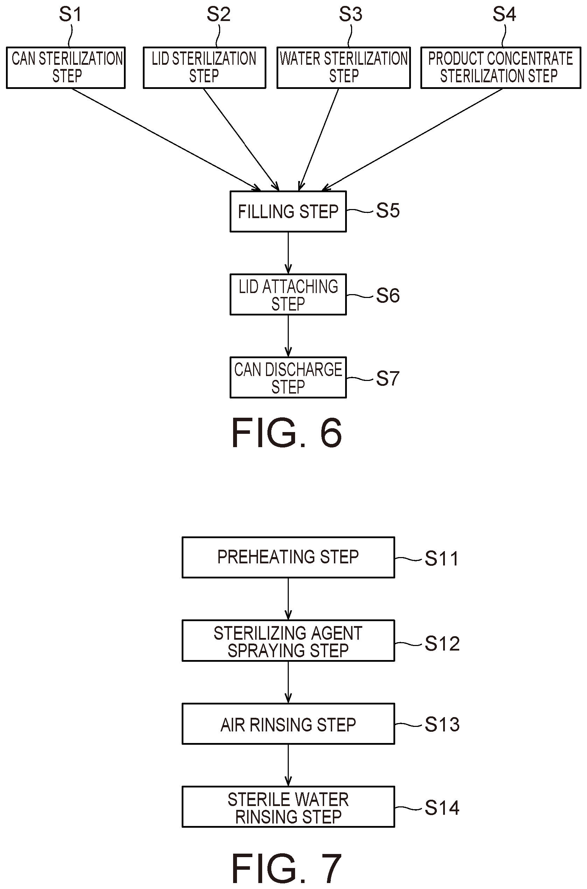

A content filling method using the above-described content filling system 10 (Fig. 1) will be described with reference to Figs. 6 and 7.

In a can sterilization step (S1 in Fig. 6), sterilization treatment using a hydrogen peroxide aqueous solution, which is a sterilizing agent, is performed on the empty can 1 by the container sterilization apparatus 20. In the sterilization treatment, initially, the can 1 is conveyed from the depalletizer (not shown). The can 1 conveyed from the depalletizer passes through a foreign matter removal device (not shown). The can 1 that has passed through the foreign matter removal device is heated in the first heating unit 21 (preheating step, S11 in Fig. 7). In the preheating step, the can 1 conveyed to the first heating unit 21 is heated to, for example, a temperature higher than or equal to 40°C and lower than or equal to 100°C by heated air from the first hot air nozzles 21a and 21b. In the preheating step, hot air is blown into the can 1 using the first hot air nozzle 21a by utilizing the thermal conductivity of the can 1 (for example, aluminum can). A heating wheel (not shown) where the preheating step is performed may have a tunnel-shaped portion through which the can 1 passes. Since the portion through which the can 1 passes has a tunnel shape, heat loss can be suppressed, and the can 1 is uniformly heated in a short time. Hot air blown from the single first hot air nozzle 21a may have a temperature higher than or equal to 120°C and lower than or equal to 200°C, a blowing time of longer than or equal to one second and shorter than or equal to three seconds, and a flow rate of higher than or equal to 200 L/min and lower than or equal to 500 L/min. The heated can 1 is conveyed to the first sterilizing agent spraying unit 22.

Next, in the first sterilizing agent spraying unit 22, a sterilizing agent is sprayed onto the can 1 (sterilizing agent spraying step, S12 in Fig. 7). In the sterilizing agent spraying step, the sterilizing agent may be a gas or mist obtained by once vaporizing a 35 wt% hydrogen peroxide aqueous solution at or above its boiling point. The gas or mist of the hydrogen peroxide aqueous solution adheres to the inner and outer surfaces of the can 1 to sterilize the inner and outer surfaces of the can 1. By using thermo-resistant yeast as the indicator microorganism, the amount of hydrogen peroxide adhering to the can can be reduced.

The can 1 of which the inner and outer surfaces have been sterilized is conveyed to the first air rinsing unit 23. When sterile heated air or room-temperature air is supplied to the can 1 in the first air rinsing unit 23, hydrogen peroxide is activated, and foreign matter, hydrogen peroxide, and the like, are removed from the can 1 (air rinsing step, S13 in Fig. 7). In the air rinsing step, sterile heated air or room-temperature air blown from the single first air rinsing nozzle 23a may have a temperature higher than or equal to 120°C and lower than or equal to 200°C. The blowing time may be longer than or equal to one second and shorter than or equal to three seconds, or may be longer than or equal to two seconds and shorter than or equal to six seconds. In the preheating step, the blowing time may be longer than or equal to the blowing time of hot air blown from the first hot air nozzle 21a, and may be longer than or equal to twice the blowing time of hot air blown from the first hot air nozzle 21a. The flow rate may be higher than or equal to 200 L/min and lower than or equal to 500 L/min. In the air rinsing step, if necessary, a low-concentration condensed mist of hydrogen peroxide may be mixed with sterile heated air or sterile air at room temperature. In this case, hydrogen peroxide is gasified by sterile air. Then, in the air rinsing step, gasified hydrogen peroxide may be supplied to the can 1.

After the air rinsing step, the can 1 is conveyed to the first sterile water rinsing unit 24. In the first sterile water rinsing unit 24, the can 1 is cleaned with sterile water (sterile water rinsing step, S14 in Fig. 7). By cleaning the can 1 with sterile water, hydrogen peroxide adhering to the can 1 is washed away, and foreign matter is removed.

After that, the can 1 is conveyed to the filling apparatus 70.

In a lid sterilization step (S2 in Fig. 6), sterilization treatment using a hydrogen peroxide aqueous solution, which is a sterilizing agent, is performed on the lid 2 by the lid sterilization apparatus 30. In the sterilization treatment, as in the case of the can 1, initially, the lid 2 is heated (preheating step, S11 in Fig. 7). In the preheating step, the lid 2 is conveyed to the second heating unit 31. The lid 2 conveyed to the second heating unit 31 is heated to, for example, a temperature higher than or equal to 40°C and lower than or equal to 100°C by heated air from the second hot air nozzles 31a. The heated lid 2 is conveyed to the second sterilizing agent spraying unit 32.

Subsequently, as in the case of the can 1, a sterilizing agent is sprayed onto the lid 2 (sterilizing agent spraying step, S12 in Fig. 7). In the sterilizing agent spraying step, the sterilizing agent may be a gas or a mist of a hydrogen peroxide aqueous solution, and the gas or the mist of the hydrogen peroxide aqueous solution adheres to the inner and outer surfaces of the lid 2 to sterilize the inner and outer surfaces of the lid 2. Subsequently, the lid 2 of which the inner and outer surfaces have been sterilized is conveyed to the second air rinsing unit 33.

When sterile heated air or room-temperature air is supplied to the lid 2 in the second air rinsing unit 33 as in the case of the can 1, hydrogen peroxide is activated, and foreign matter, hydrogen peroxide, and the like, are removed from the lid 2 (air rinsing step, S13 in Fig. 7). In the air rinsing step, gasified hydrogen peroxide may be supplied to the lid 2.

After the air rinsing step, the lid 2 is conveyed to the second sterile water rinsing unit 34. Then, as in the case of the can 1, the lid 2 is cleaned with sterile water (sterile water rinsing step, S14 in Fig. 7). By cleaning the lid 2 with sterile water, hydrogen peroxide adhering to the lid 2 is washed away, and foreign matter is removed.

After that, the lid 2 is conveyed to the lid attaching apparatus 80.

In a water sterilization step (S3 in Fig. 6), water is sterilized without heating by the water sterilization line 40. In the water sterilization step, water may be sterilized by ultraviolet light in the water sterilization line 40.

In a product concentrate sterilization step (S4 in Fig. 6), a product concentrate is sterilized by heating by the concentrate sterilization line 60.

The water sterilized in the water sterilization step and the product concentrate sterilized in the product concentrate sterilization step are mixed at the intersection of the pipe of the water sterilization line 40 with the pipe of the concentrate sterilization line 60.

In a filling step (S5 in Fig. 6), the sterilized can 1 is filled with the water and the product concentrate by the filling apparatus 70. In the filling apparatus 70, the contents produced from the water and the product concentrate are filled into the can 1.

Subsequently, the can 1 filled with the contents is conveyed to the lid attaching apparatus 80.

In a lid attaching step (S6 in Fig. 6), the can 1 filled with the water and the product concentrate (contents) is sealed with the sterilized lid 2 by the lid attaching apparatus 80. In the lid attaching apparatus 80, the sterilized lid 2 is attached to the flange 1a of the can 1 conveyed from the filling apparatus 70. By attaching the sterilized lid 2 to the flange 1a of the can 1, the can 1 is sealed, and the product can 1A is obtained. When the can 1 is sealed with the lid 2, nitrogen filtered through a sterilizing filter may be supplied to the head space of the can 1. Nitrogen gas may be supplied to the head space after being filtered through the sterilizing filter having a pore size of greater than or equal to 0.2 µm and less than or equal to 0.45 µm.

In a can discharge step (S7 in Fig. 6), the product can 1A is conveyed from the lid attaching apparatus 80 to the product can discharge unit 11 and discharged to the outside of the content filling system 10. Then, the product can 1A is warmed by the can warmer 12 and conveyed to a packaging line (not shown) and packaged. In the present embodiment, existing pasteurizers become unnecessary, a reduction in carbon dioxide emissions, a reduction in water and steam energy, and a reduction in the weight of the can can be achieved.

The above-described can sterilization step, filling step, lid attaching step, and can discharge step are performed within an aseptic atmosphere enclosed by the first heating chamber 90a, the first sterilizing agent spraying chamber 90b, the first air rinsing chamber 90c, the first sterile water rinsing chamber 90d, the filling chamber 90e, the lid attaching chamber 90f, and the outlet chamber 90g, that is, under an aseptic environment. The lid sterilization step is performed within an aseptic atmosphere enclosed by the second heating chamber 90h, the second sterilizing agent spraying chamber 90i, the second air rinsing chamber 90j, and the second sterile water rinsing chamber 90k, that is, under an aseptic environment. In this case, the chambers 90a to 90k are subjected to sterilization treatment in advance by, for example, spraying hydrogen peroxide or peracetic acid (higher than or equal to 40°C and lower than or equal to 80°C), spraying caustic soda (higher than or equal to 50°C and lower than or equal to 100°C), or spraying heated water (higher than or equal to 60°C and lower than or equal to 100°C). After the chambers are subjected to sterilization treatment, the chambers are dried with sterile heated air, and maintained in a positive pressure state by supplying sterile air at room temperature.

The water sterilization line 40 and the concentrate sterilization line 60 are cleaned (CIP) in advance using a cleaning agent containing an alkaline agent such as caustic soda or an acidic agent such as nitric acid, at higher than or equal to about 60°C and lower than or equal to about 100°C (higher than or equal to 0.1% and lower than or equal to 5%). Then, the channel is rinsed with sterile water after cleaning, then water is circulated through the pipe lines of the circulation systems 44A and 69A, and the systems are placed in a standby operation state under aseptic conditions. A sterile state is maintained by supplying sterile air to a secondary-side channel of the second water tank 42 of the downstream water sterilization line 40, and to the channel from the storage tank 16a to the filling apparatus 70 (filling valve tip). The water sterilization line 40 and the concentrate sterilization line 60 may be sterilized (SIP) after cleaning (CIP). When the contents are an RTD beverage containing alcohol and carbon dioxide, the indicator microorganism is thermo-resistant yeast. Therefore, from the middle of cleaning (CIP) until the end of production, the water sterilization line 40 and the like may be rinsed with sterile water sterilized without heating while the water sterilization line 40 and the like are maintained in a positive pressure state, and the process may proceed to production. As a result, carbon dioxide emissions and thermal energy associated with sterilization (SIP) can be reduced, and downtime between productions can also be reduced.

Here, an acidic carbonated beverage containing carbon dioxide gas is filled into the can 1 as contents. The beverage may be a ready-to-drink (RTD) beverage. The pH of beer-based beverages and the like is generally lower than 4.6. Such beer-based beverages and the like contain alcohol and carbon dioxide. Therefore, bacterial spores that are harmful in low-acid beverages are not subject to sterilization in beer-based beverages and the like. On the other hand, in beer-based beverages and the like, yeast and lactic acid bacteria that have heat resistance are the main harmful microorganisms. Here, yeast and lactic acid bacteria that are harmful in beer-based beverages and the like can be sterilized (SIP) simultaneously with the above-described cleaning (CIP) (cleaning and sterilization in place (CSIP)). Therefore, in a subsequent filling step, when these contents are filled, separate sterilization after the above-described cleaning (CIP) cleaning is not needed.

Preferably, the production (conveyance) speed of the can 1 in the content filling system 10 is higher than or equal to 100 cpm and lower than or equal to 2500 cpm. Here, cpm (can per minute) refers to the conveyance speed of the can 1 per minute.

As described above, according to the present embodiment, the content filling system 10 includes the container sterilization apparatus 20 that sterilizes the can 1, the lid sterilization apparatus 30 that sterilizes the lid 2 for sealing the can 1, the water sterilization line 40 that sterilizes water without heating, the concentrate sterilization line 60 that sterilizes a product concentrate, the filling apparatus 70 that is connected to each of the water sterilization line 40 and the concentrate sterilization line 60 and that fills the sterilized can 1 with the water and the product concentrate, and the lid attaching apparatus 80 that seals, with the sterilized lid 2, the can 1 filled with the water and the product concentrate. As a result, carbon dioxide emissions during production of the contents can be reduced as compared with a case in which the product concentrate is diluted with sterile water prepared using a sterilizer that sterilizes water by heating. Therefore, carbon dioxide emissions from the content filling system 10 can be reduced.

According to the present embodiment, the product can 1A can be manufactured by using the can 1, lid 2, water, and product concentrate sterilized in advance. Therefore, a sterilization step of sterilizing the product can 1A (so-called post-sterilization) after the contents are filled into the can 1 can be omitted. On the other hand, in general, when a carbonated product (for example, a carbonated alcoholic beverage) is produced, the product is filled at a low temperature (about 5°C) and then sterilized for about 10 minutes using a pasteurizer such that a central temperature of the can reaches about 65°C. As a result, energy consumption increases, and carbon dioxide emissions from the filling system also increase. When sterilization is performed such that the central temperature of the can reaches about 65°C, the internal pressure of the can increases, so it may be difficult to manufacture a so-called high-gas product. In an existing filling system, since a pasteurizer is installed, the filling system may become large in size. In an existing filling system, since sterilization is performed using a pasteurizer, fruit juice in contents may degrade due to heat. Furthermore, in an existing filling system, since sterilization is performed using a pasteurizer, the pressure inside the can increases. For this reason, it is sometimes difficult to reduce the weight of the can. In particular, for a so-called high-gas product with a high proportion of carbon dioxide in the contents, it is highly likely that reducing the weight of the can is difficult.

In contrast, in the present embodiment, since the product can 1A can be manufactured by using the can 1, lid 2, water, and product concentrate sterilized in advance, the sterilization step of sterilizing the product can 1A after the contents are filled into the can 1 can be omitted. Therefore, energy savings can be achieved, and carbon dioxide emissions from the content filling system 10 can be reduced. In this case, the temperature of the product can 1A is increased not to about 65°C, which is a sterilization temperature in an existing pasteurizer, but to about 30°C, which is a warming temperature by the can warmer 12. As a result, an increase in the internal pressure of the can 1 is suppressed, so a so-called high-gas product can also be manufactured. In the content filling system 10 according to the present embodiment, even when a carbonated product is manufactured, installation of a pasteurizer is unnecessary, so the size of the content filling system 10 can be reduced. Furthermore, in the content filling system 10 according to the present embodiment, since the sterilization step of sterilizing the product can 1A can be omitted, the degradation of fruit juice in the contents can be suppressed, and the can 1 and the lid 2 can be reduced in weight. In addition, with the content filling system 10 according to the present embodiment, since the container to be filled with the contents does not need to have high heat resistance and high pressure resistance, a PET can in which the body portion of the can is replaced from metal to resin (PET) may be used. When a PET can is used, the PET can does not need to have high heat resistance and high pressure resistance, so the PET can can also be reduced in weight.

In the above-described present embodiment, an example in which the container sterilization apparatus 20 sterilizes the can 1 with a sterilizing agent and the lid sterilization apparatus 30 sterilizes the lid 2 with a sterilizing agent has been described; however, the configuration is not limited thereto. For example, the container sterilization apparatus 20 may sterilize the can 1 with hot water, and the lid sterilization apparatus 30 may sterilize the lid 2 with hot water.

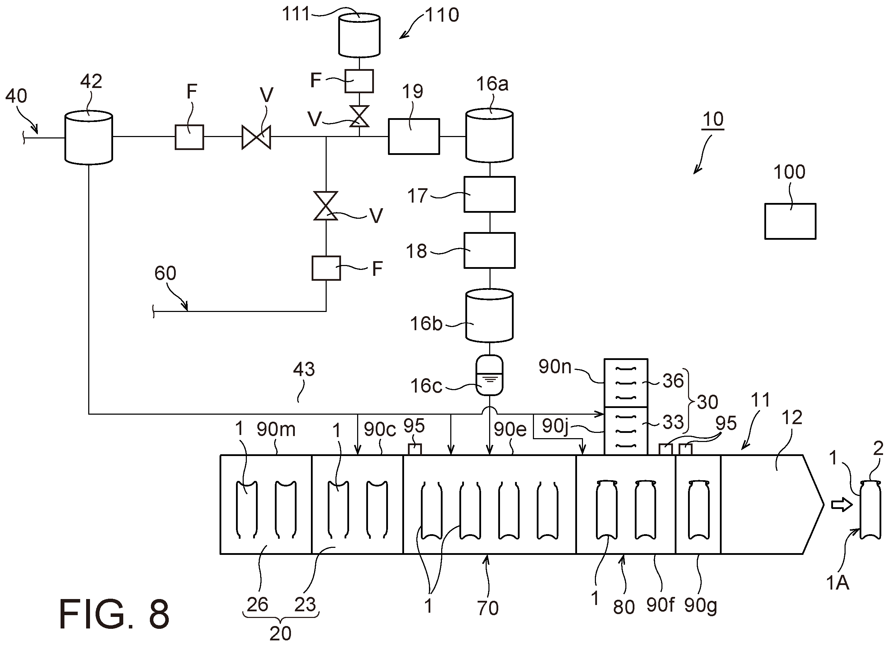

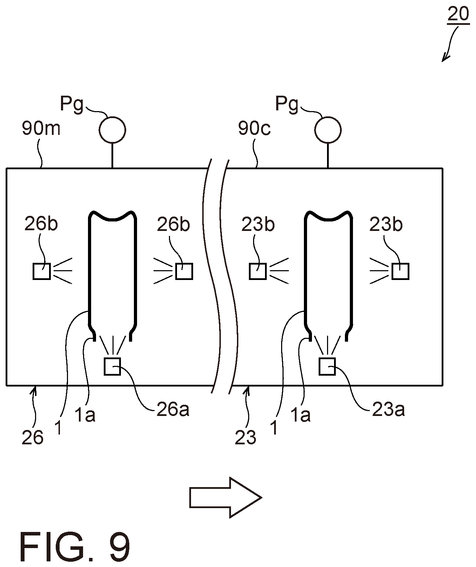

In this case, as shown in Figs. 8 and 9, the container sterilization apparatus 20 may include a first hot water supply unit 26 that supplies hot water to the can 1. The container sterilization apparatus 20 may further include the first air rinsing unit 23 that air-rinses the can 1 supplied with hot water by the first hot water supply unit 26. In other words, in this modification, the above-described first air rinsing unit 23 may be provided downstream of the first hot water supply unit 26, and the container sterilization apparatus 20 does not need to include the first heating unit 21, the first sterilizing agent spraying unit 22, and the first sterile water rinsing unit 24. In Fig. 9, the first air rinsing unit 23 is provided downstream of the first hot water supply unit 26 to also serve to remove residual water on the can 1. Alternatively, the first air rinsing unit 23 may be installed upstream of the first hot water supply unit 26 or may be installed both upstream and downstream of the first hot water supply unit 26. As shown in Fig. 9, the can 1 is conveyed in the direction of the arrow.

The first hot water supply unit 26 is a portion that sterilizes the can 1 by spraying hot water onto the can 1. The first hot water supply unit 26 is configured to spray hot water while conveying the can 1. In this case, the can 1 may be conveyed in a state where the flange 1a, to which the lid 2 is to be attached, faces downward. As shown in Fig. 9, the first hot water supply unit 26 includes first hot water nozzles 26a and 26b that spray hot water onto the can 1 being conveyed. Among these, the first hot water nozzle 26a is a nozzle for spraying hot water onto the inner surface of the can 1. The first hot water nozzle 26a may be inserted into the can 1 or may remain outside the can 1. The first hot water nozzle 26b is nozzle for spraying hot water onto the outer surface of the can 1.

The temperature of hot water supplied by the first hot water supply unit 26 may be higher than or equal to 60°C and lower than or equal to 100°C, and may be higher than or equal to 70°C and lower than or equal to 90°C. When the temperature of hot water is higher than or equal to 60°C, the sterilization effect on the can 1 can be improved. When the temperature of hot water is lower than or equal to 100°C, thermal energy consumption can be suppressed, and carbon dioxide emissions can be reduced.

In the first hot water supply unit 26, the amount of hot water adhering to the can 1 may be greater than or equal to 0.1 mL/cm² and less than or equal to 0.3 mL/cm². When the amount of hot water adhering to the can 1 is greater than or equal to 0.1 mL/cm², the sterilization effect on the can 1 can be improved. When the amount of hot water adhering to the can 1 is less than or equal to 0.3 mL/cm², the usage of hot water can be reduced, so thermal energy consumption for sterilizing hot water can be suppressed, and carbon dioxide emissions can be reduced.

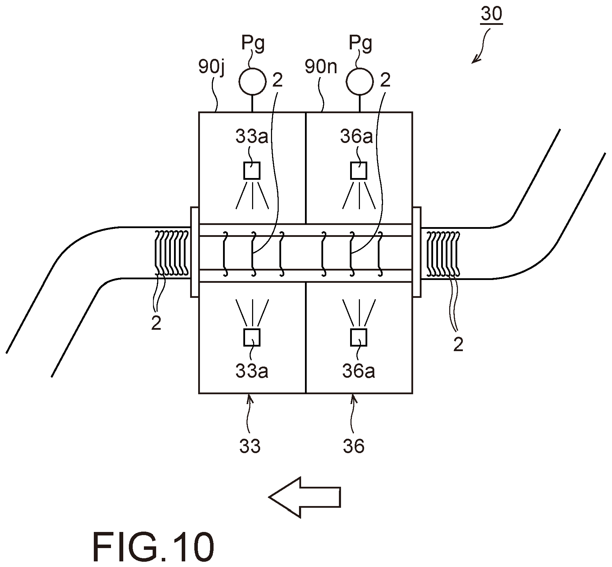

As shown in Figs. 8 and 10, the lid sterilization apparatus 30 may include a second hot water supply unit 36 that supplies hot water to the lid 2. The lid sterilization apparatus 30 may further include the second air rinsing unit 33 that air-rinses, with sterile air, the lid 2 supplied with hot water by the second hot water supply unit 36. In other words, in this modification, the above-described second air rinsing unit 33 may be provided downstream of the second hot water supply unit 36, and the lid sterilization apparatus 30 does not need to include the second heating unit 31, the second sterilizing agent spraying unit 32, and the second sterile water rinsing unit 34. In Fig. 10, the second air rinsing unit 33 is provided downstream of the second hot water supply unit 36 to also serve to remove residual water on the lid 2. Alternatively, the second air rinsing unit 33 may be installed upstream of the second hot water supply unit 36 or may be installed both upstream and downstream of the second hot water supply unit 36. As shown in Fig. 10, the lid 2 is conveyed in the direction of the arrow.

The second hot water supply unit 36 is a portion that sterilizes the lid 2 by spraying hot water onto the lid 2. The second hot water supply unit 36 is configured to spray hot water while conveying the lid 2. As shown in Fig. 10, the lid 2 may be conveyed by the guide 35, such as a so-called screw chute, such that a spacing is provided between adjacent lids 2. A spacing may be provided between adjacent lids 2 by vibrating the guide 35. Then, hot water may be applied to the lid 2 in a state where a spacing is provided between adjacent lids 2. The second hot water supply unit 36 includes second hot water nozzles 36a that spray hot water onto the lid 2 being conveyed. At each second hot water nozzle 36a, the discharge pressure of hot water may be higher than or equal to 0.1 MPa. A plurality of the second hot water nozzles 36a may be used to apply hot water to the lid 2.

The temperature of hot water supplied by the second hot water supply unit 36 may be higher than or equal to 60°C and lower than or equal to 100°C, and may be higher than or equal to 70°C and lower than or equal to 90°C. When the temperature of hot water is higher than or equal to 60°C, the sterilization effect on the lid 2 can be improved. When the temperature of hot water is lower than or equal to 100°C, thermal energy consumption can be suppressed, and carbon dioxide emissions can be reduced.

In the second hot water supply unit 36, the amount of hot water adhering to the lid 2 may be greater than or equal to 0.1 mL/cm² and less than or equal to 0.3 mL/cm². When the amount of hot water adhering to the lid 2 is greater than or equal to 0.1 mL/cm², the sterilization effect on the lid 2 can be improved. When the amount of hot water adhering to the lid 2 is less than or equal to 0.3 mL/cm², the usage of hot water can be reduced, so thermal energy consumption for sterilizing hot water can be suppressed, and carbon dioxide emissions can be reduced.

In this modification, the content filling system 10 includes a first hot water supply chamber 90m, the first air rinsing chamber 90c, the filling chamber 90e, the lid attaching chamber 90f, and the outlet chamber 90g. The first hot water supply chamber 90m, the first air rinsing chamber 90c, the filling chamber 90e, the lid attaching chamber 90f, and the outlet chamber 90g are arranged in this order from the upstream side to the downstream side along the conveying direction of the can 1.

The content filling system 10 includes a second hot water supply chamber 90n and the second air rinsing chamber 90j. The second hot water supply chamber 90n, the second air rinsing chamber 90j, the lid attaching chamber 90f, and the outlet chamber 90g are arranged in this order from the upstream side to the downstream side along the conveying direction of the lid 2. Each of the chambers 90e, 90f, and 90g, to which sterile air is not supplied, may be provided with the sterile air supply device 95. Instead of air, nitrogen gas or carbon dioxide gas may be supplied to the primary side of the sterilizing filter of the chamber 90f. As a result, the oxygen concentration in the head space of the can 1 can be further reduced.

Of these, the first hot water supply chamber 90m houses the first hot water supply unit 26 (the first hot water nozzles 26a and 26b). The second hot water supply chamber 90n houses the second hot water supply unit 36 (the second hot water nozzle 36a).

In this modification, the pressure PM in the first hot water supply chamber 90m, the pressure PC in the first air rinsing chamber 90c, and the pressure PE in the filling chamber 90e may satisfy the following relationship. $0 \left(Pa\right) \leq PM \leq PC < PE$

In this case as well, the pressure PE in the filling chamber 90e is higher than the pressure PC in the first air rinsing chamber 90c. As a result, entry of air in the first air rinsing chamber 90c into the filling chamber 90e can be suppressed. Therefore, a sterile state inside the filling chamber 90e can be favorably maintained.

The pressure PE in the filling chamber 90e, the pressure PF in the lid attaching chamber 90f, the pressure PN in the second hot water supply chamber 90n, and the pressure PJ in the second air rinsing chamber 90j may satisfy the following relationship. $0 \left(Pa\right) \leq PN \leq PJ \leq PF < PE$

In this case as well, the pressure PE in the filling chamber 90e is higher than the pressure PF in the lid attaching chamber 90f. As a result, entry of air in the lid attaching chamber 90f into the filling chamber 90e can be suppressed. Therefore, a sterile state inside the filling chamber 90e can be favorably maintained. In this case, the pressure PF in the lid attaching chamber 90f is higher than or equal to the pressure PJ in the second air rinsing chamber 90j. As a result, entry of air in the second air rinsing chamber 90j into the lid attaching chamber 90f can be suppressed. Therefore, a sterile state inside the lid attaching chamber 90f can be favorably maintained.

According to this modification, the container sterilization apparatus 20 includes the first hot water supply unit 26 that supplies hot water to the can 1. The temperature of hot water supplied by the first hot water supply unit 26 is higher than or equal to 60°C and lower than or equal to 100°C. Furthermore, in the first hot water supply unit 26, the amount of hot water adhering to the can 1 is greater than or equal to 0.1 mL/cm² and less than or equal to 0.3 mL/cm². According to this modification, the lid sterilization apparatus 30 includes the second hot water supply unit 36 that supplies hot water to the lid 2. The temperature of hot water supplied by the second hot water supply unit 36 is higher than or equal to 60°C and lower than or equal to 100°C. Furthermore, in the second hot water supply unit 36, the amount of hot water adhering to the lid 2 is greater than or equal to 0.1 mL/cm² and less than or equal to 0.3 mL/cm². As a result, the can 1 and the lid 2 can be sterilized with hot water without using a sterilizing agent. Therefore, sterilization costs for the can 1 and the lid 2 can be reduced. Here, as described above, when the contents are beer-based beverages and the like, bacterial spores that are harmful in low-acid beverages are not subject to sterilization. Therefore, even when hot water is used without using a sterilizing agent, a sufficient sterilization effect can be obtained.

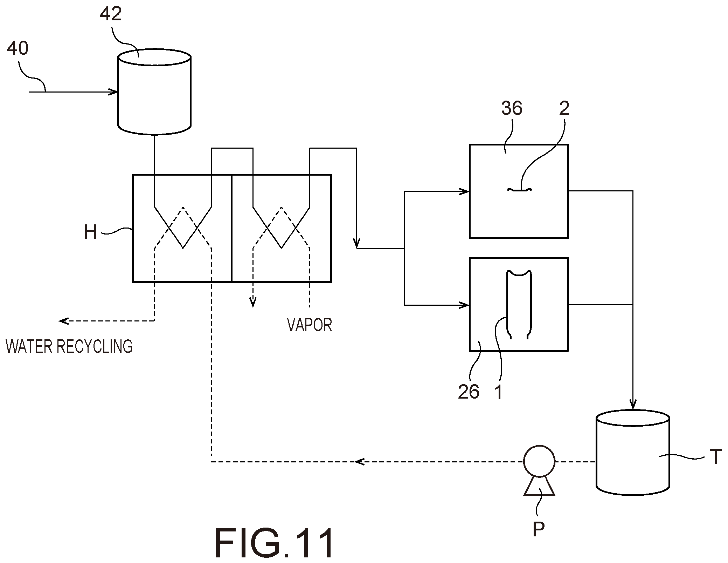

In the case of this modification, as shown in Fig. 11, heat of the hot water may be recovered. As a result, carbon dioxide emissions caused by hot water can be reduced.

When heat of hot water is recovered, specifically, sterile water sterilized without heating by the water sterilization line 40 is initially heated by a heat exchanger H to a temperature higher than or equal to 60°C and lower than or equal to 100°C as shown by the solid line in Fig. 11. At this time, sterile water is supplied to the heat exchanger H, for example, at a temperature higher than or equal to 15°C and lower than or equal to 25°C (for example, 20°C). Then, sterile water is heated to a temperature higher than or equal to 50°C and lower than or equal to 80°C (for example, 65°C) by exchanging heat with hot water recovered into a tank T (described later). The hot water that has exchanged heat with the sterile water is cooled from a temperature higher than or equal to 50°C and lower than or equal to 80°C (for example, 65°C) to a temperature higher than or equal to 25°C and lower than or equal to 35°C (for example, 30°C). Subsequently, the sterile water is heated to a temperature higher than or equal to 70°C and lower than or equal to 80°C (for example, 75°C) by exchanging heat with vapor supplied to the heat exchanger H.

After that, the heated hot water is supplied to the first hot water supply unit 26 and the second hot water supply unit 36, and is used to sterilize the can 1 and/or the lid 2.

Subsequently, the hot water used to sterilize the can 1 and/or the lid 2 is recovered into the tank T. Then, the hot water recovered in the tank T is returned to a medium side of the heat exchanger H. In this way, heat of the hot water is recovered. As a result, thermal energy (and/or carbon dioxide) can be significantly reduced. The hot water after heat exchange may be reused by returning the hot water after heat exchange to the first water tank 41. In this case, foreign matter may enter the hot water from the can 1, the chambers, the pipes, and the like. In this way, even when foreign matter enters the hot water to be reused, the foreign matter in the hot water is removed by the foreign matter removal filter 51 of the water sterilization line 40.

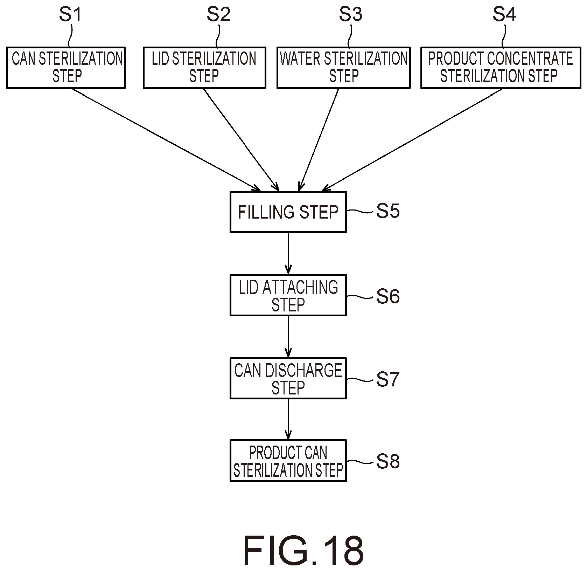

In the above-described present embodiment, an example in which the sterilization step of sterilizing the product can 1A is omitted has been described; however, the configuration is not limited thereto. For example, after the above-described can discharge step (S7 in Fig. 6), the can 1 sealed with the lid 2 may be sterilized. In other words, as shown in Fig. 18, after the above-described can discharge step (S7 in Fig. 6, and S7 in Fig. 18), the product can 1A may be sterilized (product can sterilization step, S8 in Fig. 18). The product can 1A is, for example, a high-gas product having a gas volume of greater than or equal to 3.0. The contents are, for example, an alcoholic beverage. By increasing the gas volume of the product can 1A and/or the alcohol content of the contents, the sterilization temperature can be lowered as compared with when the gas volume and/or the alcohol content are not high. According to the present embodiment, since sterilization of the contents is performed before filling, a sterilization time after filling can be shortened. For the product can 1A, which is a high-gas product, by using the content filling system 10 according to the present embodiment, sterilization in the sterilization step of sterilizing the product can 1A can be performed at a low temperature and in a short time.

In the product can sterilization step, a sterilization time may be longer than or equal to 0.5 minutes and shorter than or equal to 10 minutes. In the product can sterilization step, a sterilization temperature may be higher than or equal to 50°C and lower than or equal to 60°C. Since the sterilization time is shorter than or equal to 10 minutes and the sterilization temperature is lower than or equal to 60°C, sterilization can be performed at a low temperature and in a short time.

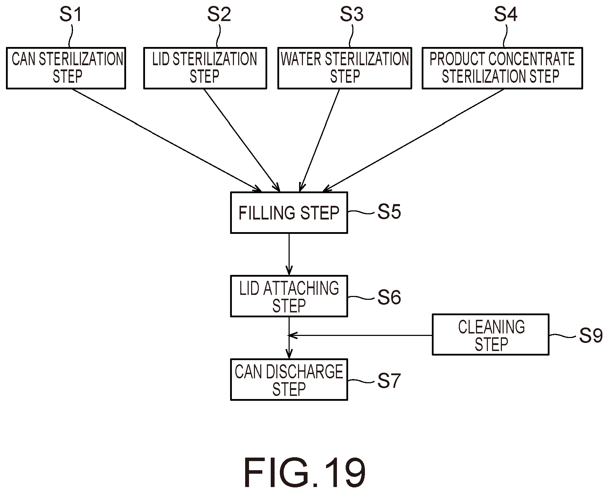

In the above-described present embodiment, an example in which the can discharge step (S7 in Fig. 6) is performed after the lid attaching step (S6 in Fig. 6) has been described. In this case, for example, as shown in Fig. 19, the lid attaching apparatus (seamer) 80 may be cleaned after the lid attaching step (S6 in Fig. 19) (cleaning step, S9 in Fig. 19).

Here, in a filling system that fills an RTD beverage, the can 1 filled with contents is conveyed at high speed. The turning speed of the lid 2 in the lid attaching apparatus 80 may be higher than or equal to 100 cpm and lower than or equal to 2500 cpm, and may be higher than or equal to 1000 cpm and lower than or equal to 2000 cpm. When the can 1 is conveyed at high speed, the contents may spill out of the can 1 in the lid attaching apparatus 80. Spilled contents may dry and become a biofilm (a collection of microorganisms). In contrast, by cleaning the lid attaching apparatus (seamer) 80 after the lid attaching step (S6 in Fig. 19), formation of a biofilm can be suppressed.

When the lid attaching apparatus (seamer) 80 is cleaned, the lid attaching apparatus 80 may be cleaned with water sterilized without heating by the water sterilization line 40. As a result, carbon dioxide emissions from the content filling system 10 can be reduced as compared with a case where the lid attaching apparatus 80 is cleaned with sterile water prepared by using a sterilizer that sterilizes water by heating. When the lid attaching apparatus 80 is cleaned, the control unit 100 may clean the lid attaching apparatus 80 by controlling the water sterilization line 40.

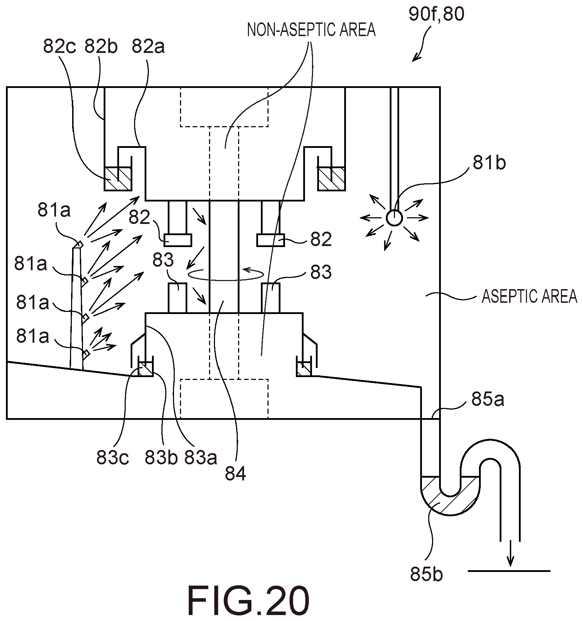

When the lid attaching apparatus (seamer) 80 is cleaned, the control unit 100 may clean the inside of the lid attaching chamber 90f that houses the lid attaching apparatus 80. When the inside of the lid attaching chamber 90f is cleaned, the control unit 100 may constantly supply water sterilized without heating to first cleaning nozzles 81a (see Fig. 20) of the lid attaching apparatus 80.

As shown in Fig. 20, the lid attaching apparatus 80 includes a plurality of seaming rolls 82 and a plurality of lifters 83 corresponding to the respective seaming rolls 82. The seaming roll 82 is a member used to seam the lid 2 onto the can 1. Generally, seaming of the can 1 is performed by two steps, that is, first seaming and second seaming. For this reason, the seaming roll 82 includes a first seaming roll (not shown) that performs first seaming and a second seaming roll (not shown) that performs second seaming, the second seaming roll being different from the first seaming roll. The lifter 83 is a member for moving the can 1 up and down while rotating and conveying the can 1.

The above-described first cleaning nozzles 81a may be configured to spray sterile water from above the seaming roll 82 to below the lifter 83. The inside of the lid attaching chamber 90f that houses the lid attaching apparatus 80 may be constantly cleaned by the first cleaning nozzles 81a. Among the arrows shown in Fig. 20, the arrows extending in a straight line represent the sprayed sterile water.

The seaming rolls 82 and lifters 83 of the lid attaching apparatus 80 each are revolvable around a rotary shaft 84. Then, a sterilizing agent pool 82c in which a sterilizing agent (peracetic acid) is stored is provided between a rotatable body 82a that revolves the seaming rolls 82 and a fixed body 82b surrounding the rotatable body 82a. A sterilizing agent pool 83c in which a sterilizing agent (peracetic acid) is stored is provided between a rotatable body 83a that revolves the lifters 83 and a fixed body 83b surrounding the rotatable body 83a. With these sterilizing agent pools 82c and 83c, a machine room (non-aseptic area) in which driving components and electrical components of the lid attaching apparatus (seamer) 80, seaming rolls 82, and lifters 83 are housed can be separated from the lid attaching chamber 90f (aseptic area or sterilization target area), so an aseptic condition within the lid attaching chamber 90f is maintained.

In addition to the first cleaning nozzles 81a, the lid attaching apparatus 80 may include a second cleaning nozzle 81b that can clean the entire lid attaching chamber 90f with sterile water. Water sprayed from the first cleaning nozzles 81a and the second cleaning nozzle 81b and used for cleaning is drained from the lid attaching chamber 90f through a drain channel 85a provided near the outlet of the lid attaching chamber 90f. To maintain the aseptic condition of the lid attaching apparatus 80 for a long period of time, the drain channel 85a may have a water seal structure 85b. An end of the drain channel 85a may be separated without being directly connected to a pressurized drain pipe. As a result, backflow of liquid in the pressurized drain pipe into the lid attaching chamber 90f can be suppressed. A pump may be installed before the water seal structure 85b such that water used for cleaning does not accumulate inside the lid attaching chamber 90f. In order to drain the water used for cleaning through the drain channel 85a, the bottom surface of the lid attaching chamber 90f may be inclined downward toward the drain channel 85a.

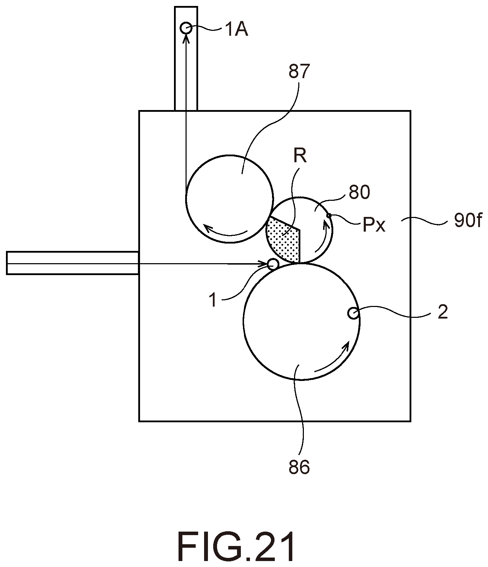

Here, as shown in Fig. 21, in the lid attaching apparatus 80, the lid 2 is supplied from a transfer wheel 86 located upstream of the lid attaching apparatus 80. The can 1 seamed by the lid attaching apparatus 80 (that is, the product can 1A) is discharged to a transfer wheel 87 located downstream of the lid attaching apparatus 80. When the lid attaching apparatus 80 and the inside of the lid attaching chamber 90f (hereinafter, also referred to as the lid attaching apparatus 80 and the like) are cleaned, a section R (the shaded area shown in Fig. 21) from the location where seaming of the can 1 is completed and the can 1 is discharged to the transfer wheel 87 until the next seaming begins may be constantly cleaned. As described above, seaming of the can 1 is performed by first seaming and second seaming. In this case, the lid attaching apparatus 80 and the like may be cleaned from a point Px where first seaming is completed.

The surface member of the lid attaching apparatus 80 may be made of SUS304 or SUS316L in consideration of corrosion resistance. When the lid attaching apparatus 80 and the like are cleaned, supply of the can 1 to the filling apparatus 70 may be temporarily stopped, and the lid attaching apparatus 80 and the like may be cleaned in a state where the lid attaching apparatus 80 is rotated at 600 cpm or lower, or 100 cpm or lower. In other words, the lid attaching apparatus 80 and the like may be cleaned in a state where the seaming rolls 82 and the lifters 83 are being driven without supplying the can 1 and the lid 2 to the lid attaching apparatus 80.

To improve the cleanability of the lid attaching apparatus 80 and the lid attaching chamber 90f, the lid attaching apparatus 80 and the lid attaching chamber 90f may be cleaned with hot water by heating sterile water produced by the water sterilization line 40. The temperature of the hot water may be higher than or equal to 40°C and lower than or equal to 100°C, and may be higher than or equal to 60°C and lower than or equal to 90°C. When hot water is produced, heat of the hot water may be recovered as described with reference to Fig. 11.

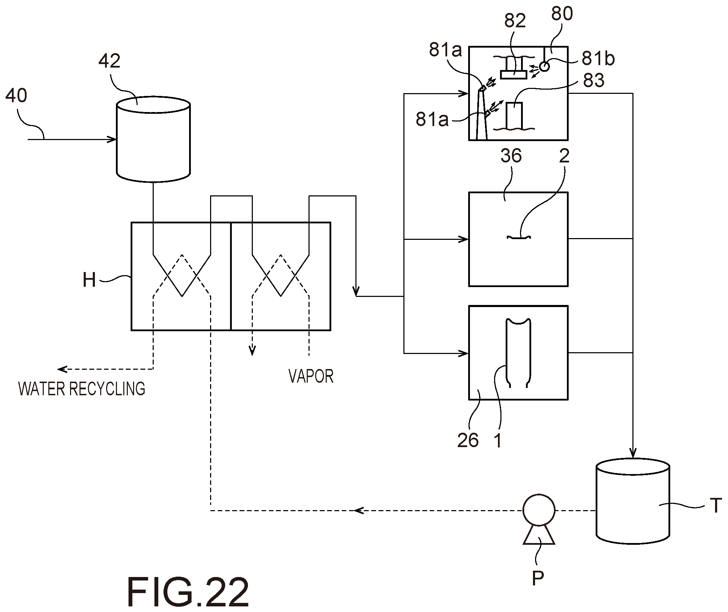

In other words, when heat of hot water is recovered, specifically, sterile water sterilized without heating by the water sterilization line 40 is initially heated by the heat exchanger H to a temperature higher than or equal to 60°C and lower than or equal to 100°C as shown by the solid line in Fig. 22. At this time, sterile water is supplied to the heat exchanger H, for example, at a temperature higher than or equal to 15°C and lower than or equal to 25°C (for example, 20°C). Then, sterile water is heated to a temperature higher than or equal to 50°C and lower than or equal to 80°C (for example, 65°C) by exchanging heat with hot water recovered into a tank T (described later). The hot water that has exchanged heat with the sterile water is cooled from a temperature higher than or equal to 50°C and lower than or equal to 80°C (for example, 65°C) to a temperature higher than or equal to 25°C and lower than or equal to 35°C (for example, 30°C). Subsequently, the sterile water is heated to a temperature higher than or equal to 70°C and lower than or equal to 80°C (for example, 75°C) by exchanging heat with vapor supplied to the heat exchanger H.

After that, the heated hot water is supplied to the first cleaning nozzles 81a and the second cleaning nozzle 81b of the lid attaching apparatus 80, and is used to clean the lid attaching apparatus 80 and the inside of the lid attaching chamber 90f.

Subsequently, the hot water used to clean the lid attaching apparatus 80 and the like is recovered into the tank T. Then, the hot water recovered in the tank T is returned to a medium side of the heat exchanger H. In this way, heat of the hot water is recovered.

In the illustrated example, the cleaning step is performed between the lid attaching step (S6 in Fig. 19) and the can discharge step (S7 in Fig. 19); however, the configuration is not limited thereto. For example, the cleaning step may be performed after the discharge step. The cleaning step may be performed between the lid attaching step and the can discharge step, and after the discharge step.

Furthermore, in the above-described present embodiment, an example in which the sterilizing agent used to sterilize the can 1 and the lid 2 is hydrogen peroxide has been described; however, the configuration is not limited thereto. For example, the sterilizing agent used to sterilize the can 1 and the lid 2 may be slightly acidic hypochlorous acid water (a pH of 5 to 6.5). The effective chlorine concentration of the slightly acidic hypochlorous acid water may be higher than or equal to 1 ppm and lower than or equal to 100 ppm, and may be higher than or equal to 10 ppm and lower than or equal to 60 ppm. The temperature of the slightly acidic hypochlorous acid water may be higher than or equal to 20°C and lower than or equal to 90°C, and may be higher than or equal to 30°C and lower than or equal to 80°C. A cleaning time with the slightly acidic hypochlorous acid water may be longer than or equal to 0.5 seconds and shorter than or equal to 10 seconds, and may be longer than or equal to one second and shorter than or equal to five seconds.

When slightly acidic hypochlorous acid water is used as a sterilizing agent for sterilizing the can 1 and the lid 2, the can 1 is initially conveyed from a depalletizer (not shown) in the can sterilization step (see S1 in Fig. 6). The can 1 conveyed from the depalletizer passes through a foreign matter removal device (not shown). The can 1 that has passed through the foreign matter removal device is conveyed to the first sterilizing agent spraying unit 22. Depending on a foreign matter contamination condition of the can 1, the can 1 may pass without passing through the foreign matter removal device.

Next, in the first sterilizing agent spraying unit 22, the sterilizing agent is sprayed onto the can 1 (sterilizing agent spraying step, see S12 in Fig. 7). In the sterilizing agent spraying step, the slightly acidic hypochlorous acid water adheres to the inner and outer surfaces of the can 1 to sterilize the inner and outer surfaces of the can 1.

The can 1 of which the inner and outer surfaces have been sterilized is conveyed to the first sterile water rinsing unit 24. In the first sterile water rinsing unit 24, the can 1 is cleaned with sterile water (sterile water rinsing step, see S14 in Fig. 7). When the can 1 is cleaned with sterile water, the slightly acidic hypochlorous acid water adhering to the can 1 is washed away, and foreign matter is removed. In the sterile water rinsing step, the temperature of sterile water may be higher than or equal to 10°C and lower than or equal to 80°C, and may be higher than or equal to 20°C and lower than or equal to 50°C. In the sterile water rinsing step, a cleaning time with the sterile water may be longer than or equal to 0.5 seconds and shorter than or equal to 10 seconds, and may be longer than or equal to one second and shorter than or equal to five seconds. The temperature of sterile water rinsing may be lower than the temperature of the slightly acidic hypochlorous acid water, and the can 1 may be cooled by the sterile water. As a result, foaming when a carbonated beverage is filled can be reduced. The inner and outer surfaces of the can 1 may be air-rinsed by the first air rinsing unit 23 before sterile water rinsing to reduce the usage of the sterile water and a cleaning time with the sterile water thereafter.

After that, the can 1 is conveyed to the filling apparatus 70.

In the lid sterilization step (see S2 in Fig. 6), sterilization treatment using slightly acidic hypochlorous acid water, which is a sterilizing agent, is performed on the lid 2 by the lid sterilization apparatus 30. In the sterilization treatment, as in the case of the can 1, the lid 2 is conveyed to the second sterilizing agent spraying unit 32.

Subsequently, a sterilizing agent is sprayed onto the lid 2 (sterilizing agent spraying step, see S12 in Fig. 7). In the sterilizing agent spraying step, the slightly acidic hypochlorous acid water adheres to the inner and outer surfaces of the lid 2 to sterilize the inner and outer surfaces of the lid 2.

The lid 2 of which the inner and outer surfaces have been sterilized is conveyed to the second sterile water rinsing unit 34. Then, as in the case of the can 1, the lid 2 is cleaned with sterile water (sterile water rinsing step, see S14 in Fig. 7). By cleaning the lid 2 with sterile water, hydrogen peroxide adhering to the lid 2 is washed away, and foreign matter is removed. In the sterile water rinsing step, the temperature of sterile water may be higher than or equal to 10°C and lower than or equal to 80°C, and may be higher than or equal to 20°C and lower than or equal to 50°C. In the sterile water rinsing step, a cleaning time with the sterile water may be longer than or equal to 0.5 seconds and shorter than or equal to 10 seconds, and may be longer than or equal to one second and shorter than or equal to five seconds. The lid 2 may be air-rinsed by the second air rinsing unit 33 before sterile water rinsing to reduce the usage of the sterile water and a cleaning time with the sterile water thereafter.

After that, the lid 2 is conveyed to the lid attaching apparatus 80.

In this way, the can 1 and the lid 2 may be sterilized using slightly acidic hypochlorous acid water.

The plurality of component elements described in the embodiment and the modifications may be combined as needed. Alternatively, some component elements may be deleted from all the component elements described in the embodiment and the modifications.

## Claims

1. A content filling system comprising:
a container sterilization apparatus configured to sterilize a can;
a lid sterilization apparatus configured to sterilize a lid used to seal the can;
a water sterilization line configured to sterilize water without heating;
a concentrate sterilization line configured to sterilize a product concentrate;
a filling apparatus connected to the water sterilization line and the concentrate sterilization line, the filling apparatus being configured to fill the sterilized can with the water and the product concentrate; and
a lid attaching apparatus configured to seal, with the sterilized lid, the can filled with the water and the product concentrate.

2. The content filling system according to claim 1, wherein the water sterilization line is configured to sterilize the water by at least one of ultraviolet light and filtration.

3. The content filling system according to claim 1, wherein the concentrate sterilization line is configured to sterilize the product concentrate by at least one of heating, storage, and filtration.

4. The content filling system according to claim 1, further comprising a base alcohol line configured to add alcohol to the sterilized water and the sterilized product concentrate.

5. The content filling system according to claim 1, wherein
the container sterilization apparatus includes
a first sterilizing agent spraying unit configured to spray a sterilizing agent onto the can, and
a first air rinsing unit configured to air-rinse the can onto which the sterilizing agent has been sprayed by the first sterilizing agent spraying unit.

6. The content filling system according to claim 5, wherein
the sterilizing agent is a gas or a mist of a hydrogen peroxide aqueous solution, and
in the first sterilizing agent spraying unit, an amount of the sterilizing agent adhering to the can is greater than or equal to 0.01 µL/cm² and less than or equal to 0.4 µL/cm².

7. The content filling system according to claim 6, wherein
the sterilizing agent is a gas or a mist of a hydrogen peroxide aqueous solution,
in the first air rinsing unit, sterile air at 70°C to 200°C is blown onto the can, and
after the sterile air has been blown onto the can, an amount of the sterilizing agent adhering to the can is greater than or equal to 0.00001 µL/cm² and less than or equal to 0.01 µL/cm².

8. The content filling system according to claim 5, wherein the container sterilization apparatus further includes a first heating unit provided upstream of the first sterilizing agent spraying unit, the first heating unit being configured to heat the can.

9. The content filling system according to claim 5, wherein the container sterilization apparatus further includes a first sterile water rinsing unit provided downstream of the first air rinsing unit.

10. The content filling system according to claim 1, wherein
the lid sterilization apparatus includes
a second sterilizing agent spraying unit configured to spray a sterilizing agent onto the lid, and
a second air rinsing unit configured to air-rinse the lid onto which the sterilizing agent has been sprayed by the second sterilizing agent spraying unit.

11. The content filling system according to claim 10, wherein
the sterilizing agent is a gas or a mist of a hydrogen peroxide aqueous solution, and
in the second sterilizing agent spraying unit, an amount of the sterilizing agent adhering to the lid is greater than or equal to 0.01 µL/cm² and less than or equal to 0.4 µL/cm².

12. The content filling system according to claim 11, wherein
the sterilizing agent is a gas or a mist of a hydrogen peroxide aqueous solution,
in the second air rinsing unit, sterile air at 70°C to 200°C is blown onto the lid, and
after the sterile air has been blown onto the lid, an amount of the sterilizing agent adhering to the lid is greater than or equal to 0.00001 µL/cm² and less than or equal to 0.01 µL/cm².

13. The content filling system according to claim 10, wherein the lid sterilization apparatus further includes a second heating unit provided upstream of the second sterilizing agent spraying unit, the second heating unit being configured to heat the lid.

14. The content filling system according to claim 10, wherein the lid sterilization apparatus further includes a second sterile water rinsing unit provided downstream of the second air rinsing unit.

15. The content filling system according to claim 1, wherein
the container sterilization apparatus includes
a first hot water supply unit configured to supply hot water to the can,
a temperature of the hot water supplied by the first hot water supply unit is higher than or equal to 70°C and lower than or equal to 90°C, and
in the first hot water supply unit, an amount of the hot water adhering to the can is greater than or equal to 0.1 mL/cm² and less than or equal to 0.3 mL/cm².

16. The content filling system according to claim 15, wherein
the container sterilization apparatus further includes
a first air rinsing unit configured to air-rinse the can to which the hot water has been supplied by the first hot water supply unit.

17. The content filling system according to claim 1, wherein
the lid sterilization apparatus includes
a second hot water supply unit configured to supply hot water to the lid,
a temperature of the hot water supplied by the second hot water supply unit is higher than or equal to 70°C and lower than or equal to 90°C, and
in the second hot water supply unit, an amount of the hot water adhering to the lid is greater than or equal to 0.1 mL/cm² and less than or equal to 0.3 mL/cm².

18. The content filling system according to claim 17, wherein
the lid sterilization apparatus further includes
a second air rinsing unit configured to air-rinse the lid to which the hot water has been supplied by the second hot water supply unit.

19. The content filling system according to claim 1, wherein the can is a seamless can.

20. The content filling system according to claim 1, wherein the can has a body portion and a bottom portion, and at least one of a recessed portion and a protruding portion is formed in the body portion.

21. The content filling system according to claim 1, wherein the can has a flange portion, a neck portion, a body portion, and a bottom portion, and the neck portion is a smooth neck.

22. The content filling system according to claim 1, wherein the can is configured to change in shape due to a pressure change upon opening.

23. The content filling system according to claim 1, wherein the lid includes aluminum, and a weight of the lid is less than or equal to 3.2 g.

24. The content filling system according to claim 1, wherein contents to be filled by the content filling system are an acidic carbonated beverage containing carbon dioxide gas.

25. The content filling system according to claim 24, wherein Brix of the contents is higher than or equal to 0% and lower than or equal to 30%.

26. The content filling system according to claim 25, wherein the contents include fruit juice.

27. The content filling system according to claim 1, wherein a gas volume in the can sealed with the lid is greater than or equal to 3.0.

28. The content filling system according to claim 1, further comprising
a control unit configured to control the water sterilization line, wherein
the control unit is configured to clean the lid attaching apparatus with water that has been sterilized without heating by the water sterilization line.

29. The content filling system according to claim 1, wherein the can includes aluminum, a weight of the can is less than or equal to 11.0 g when a capacity of the can is 350 mL, and a weight of the can is less than or equal to 14.5 g when a capacity of the can is 500 mL.

30. A manufacturing method for a contents-filled container, the manufacturing method comprising:
a can sterilization step of performing a sterilization treatment on a can by a container sterilization apparatus;
a lid sterilization step of performing a sterilization treatment on a lid by a lid sterilization apparatus;
a water sterilization step of sterilizing water without heating by a water sterilization line;
a product concentrate sterilization step of sterilizing a product concentrate by a concentrate sterilization line;
a filling step of filling the water and the product concentrate into the sterilized can by a filling apparatus; and
a lid attaching step of, by a lid attaching apparatus, sealing, with the sterilized lid, the can filled with the water and the product concentrate.

31. The manufacturing method for a contents-filled container according to claim 30, further comprising
a product can sterilization step of sterilizing the can sealed with the lid, wherein
in the product can sterilization step, a sterilization time is longer than or equal to 0.5 minutes and shorter than or equal to 10 minutes, and a sterilization temperature is higher than or equal to 50°C and lower than or equal to 60°C.

32. The manufacturing method for a contents-filled container according to claim 30, further comprising
a cleaning step of cleaning the lid attaching apparatus after the lid attaching step, wherein
in the cleaning step, the lid attaching apparatus is cleaned with water that has been sterilized without heating by the water sterilization line.
